# EUROPÄISCHE PATENTSCHRIFT

(11) **EP 2 205 597 B1**
(45) Veröffentlichungstag und Bekanntmachung des Hinweises auf die Patenterteilung: **27.03.2013**
(21) Anmeldenummer: 08804736.0
(22) Anmeldetag: 25.09.2008
(51) Int. Cl.: C07D 451/00

(54) **VERFAHREN ZUR HERSTELLUNG VON (1R,5S)-ANHYDROECGONINESTERSALZEN**
METHOD FOR PRODUCING (1R,5S) ANHYDROECGONINE ESTER SALTS
PROCÉDÉ DE PRODUCTION DE SELS DE (1R,5S)-ANHYDROECGONINESTER

(30) Priorität: 28.09.2007 DE 102007046887
(43) Veröffentlichungstag der Anmeldung: 14.07.2010
(73) Patentinhaber: Boehringer Ingelheim Pharma GmbH & Co. KG, 55216 Ingelheim am Rhein (DE)
(72) Erfinder: PUDER, Carsten H., 55216 Ingelheim Am Rhein (DE); HOELLMUELLER, Thomas, 55216 Ingelheim Am Rhein (DE)
(74) Vertreter: Simon, Elke Anna Maria
(86) Internationale Anmeldenummer: PCT/EP2008/062839
(87) Internationale Veröffentlichungsnummer: WO 2009/043793

(56) Entgegenhaltungen:
- EP-A- 1 118 674
- WO-A-2004/072071
- C. GRUNDMANN, G. OTTMANN: "Ein neuer synthetischer Weg in die Tropan-reihe" JUSTUS LIEBIGS ANNALEN DER CHEMIE, Bd. 605, 1957, Seiten 24-32, XP002513402 in der Anmeldung erwähnt
- DAVIES, H.M.L. ET AL.: "Enantioselective Synthesis of Functionalized Tropanes by Rhodium(II) Carboxylate-Catalyzed decomposition of Vinyldiazomethanes in the Presence of Pyrroles" JOURNAL OF ORGANIC CHEMISTRY, Bd. 62, 1997, Seiten 1095-1105, XP002513403
- S.P. FINDLAY: "A Preparation and Certain Properties of 2-Carbomethoxy-N-methylgranatonine" JOURNAL OF ORGANIC CHEMISTRY, Bd. 22, 1957, Seiten 391-394, XP002513404
- S.P. FINDLAY: "Concerning 2-Carbomethoxytropinone" JOURNAL OF ORANIC CHEMISTRY, Bd. 22, 1957, Seiten 1385-1394, XP002513494 in der Anmeldung erwähnt
- COSETLLO, GERARD F: "2-(3,4-Dichlorophenyl)-N-methyl-N-[2-(1-p yrrolidinyl)-1-substituted- ethyl]-acetamides: the use of conformational analysis in the development of a novel series of potent opioid kappa agonists." JOURNAL OF MEDICNAL CHEMISTRY, Bd. 34, 1991, Seiten 181-189, XP002513637

## Beschreibung

Die Erfindung betrifft ein Verfahren zur Herstellung von (1R,5S)-Anhydroecgoninestersalzen. Das erfindungsgemäße Verfahren eignet sich insbesondere für die großtechnischen Herstellung dieser Salze in hoher Enantiomerenreinheit bezüglich der (1R,5S)-Anhydroecgoninester.

Bei den der vorliegenden Erfindung zugrunde liegenden (1R,5S)-Anhydroecgoninestern handelt es sich um gut charakterisierbare Wirkstoffvorstufen mit der folgenden allgemeinen chemischen Formel **5**: 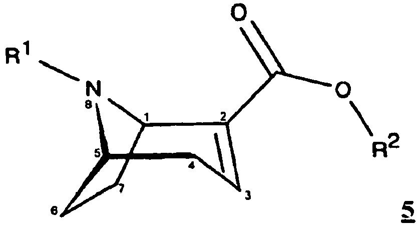 wobei
R¹ Wasserstoff, einen Alkylrest, bevorzugt Methyl, Ethyl, Propyl oder Butyl, oder eine beliebige Schutzgruppe, bevorzugt Allyl, Benzyl, Methoxybenzyl, Allyloxycarbonyl, Benzyloxycarbonyl, tert.-Butyloxycarbonyl, 9-Fluorenylmethyloxycarbonyl, Acetyl, Benzoyl oder Formyl, darstellt;
R² Alkyl, Aryl, bevorzugt Phenyl oder Naphthyl, gegebenenfalls substituiert mit ein oder mehreren Substituenten ausgewählt aus Halogen, Hydroxy, Amino, Cyano, Nitro, Trifluormethyl, Trifluormethoxy, Alkoxy, Cycloalkoxy, Alkyl, Cycloalkyl, Cycloalkylalkyl, Alkenyl und Alkinyl darstellt; bevorzugt ist R² Methyl, Ethyl, Propyl oder Butyl.

(1R,5S)-Anhydroecgoninester dienen als Ausgangsmaterial für die Herstellung von pharmazeutischen Wirkstoffen. Das 8-Azabicyclo[3.2.1]oct-2-en-System, das sich vom Tropan als Grundgerüst ableitet, stellt ein einfach ungesättigtes heterocyclisches Ringsystem dar, bei welchem das 1. und 5. C-Atom eines Piperidin-Ringes durch eine Ethylen-Gruppe miteinander verbunden sind. Beispielsweise spielen derartige Systeme als Ausgangs- oder Zwischenprodukte für pharmazeutisch aktive Tropanderivate eine Rolle. Derartige Systeme erlangen zum Beispiel Bedeutung im Zusammenhang mit potenten und selektiven Liganden des nikotinischen Acetylcholinrezeptoren (nAChR). Man erhofft sich von derartigen Liganden eine günstige Beeinflussung von bestimmten Erkrankungen, beispielsweise Demenz, wie Altersdemenz, Alzheimer Erkrankung, Parkinsonscher Erkrankung oder Hirnleistungsstörungen, wie Depressionen und Psychosen.

Aus dem Stand der Technik sind mehrere Wege bekannt geworden, wie diese (1R,5S)-Anhydroecgoninester hergestellt werden können:

Eine Möglichkeit besteht darin, von Kokain, wie beispielsweise Kokainhydrochlorid als Ausgangsmaterial auszugehen und dieses mit Alkoholat zum enantiomerenreinen (1R,5S)-Anhydroccgonincstcr umzusetzen. Diese sogenannte Kokain-Route ist zum Beispiel in der Patentanmeldung WO 96/30371 A1 beschrieben und wird in der nachfolgenden Reaktionsgleichung zur Herstellung von Anhydroecgoninethylester dargestellt:

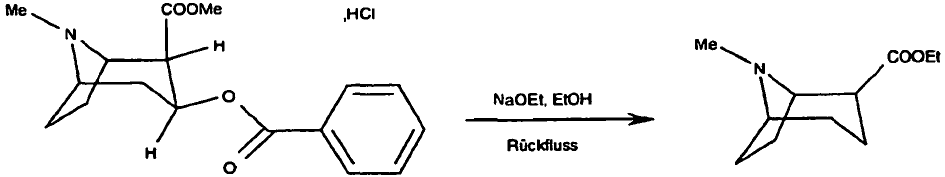

Eine Racematspaltung wird hier umgangen, indem bereits die gewünschte absolute Konfiguration als Ausgangsmaterial eingesetzt wird.

Die Nachteile, Kokain als Ausgangsmaterial für eine Synthese einzusetzen, liegen auf der Hand: Der Weltmarkt für kontrolliert, d.h. legal gewonnenes Kokain ist klein und die Zahl der Anbieter gering. So sind die Preise für z.B. Kokainhydrochlorid sehr hoch und damit ist die Synthese von (1R,5S)-Anhydroecgoninestern über die Kokain-Route entsprechend teuer. Weiterhin fällt Kokain unter das Betäubungsmittelgesetz, das den generellen Umgang mit Betäubungsmitteln regelt, so dass es für den Erwerb einer Sondererlaubnis des Bundesinstituts für Arzneimittel und Medizinprodukte (BfArM) bedarf.

Es ist demnach eine Synthese, insbesondere Totalsynthese, des Anhydroecgonin-Systems vorzuziehen, die nicht auf Kokain als Ausgangsmaterial zurückgreifen muss.

Beispielsweise wird in S. P. Findlay, J. Org. Chem. 1957, 22, 1385-1394, eine Totalsynthese des Pikrats von racemischen Anhydroecgoninmethylester beschrieben, wobei die Herstellung der Vorstufe 2-Carbomethoxytropinon ausgehend von Ketoglutarsäureanhydrid, Methylamin und Succindialdehyd durchgeführt wird. Eine Racematspaltung des 2-Carbomethoxytropinons mittels (2R,3R)-Weinsäure (L-Weinsäure) über das Hydrogentartrat wird beschrieben.

Weiterhin beschreibt die WO 2004/072071 A1 die Reduktion einer Carbomethoxytropinon-Base mittels Natriumborhydrid und die Umsetzung mit Natriumethylat in Ethylester zum Anhydroecgoninethylester. In der WO 2004/072071 A1 wird jedoch nichts über die Enantiomeren-Reinheit der eingesetzten Base und des daraus gewonnenen Anhydroecgoninethylesters ausgesagt.

Nachteilig an den geschilderten Syntheseverfahren aus dem Stand der Technik ist, dass diese nicht auf großtechnischen Maßstab ausgelegt sind und die besonderen Anforderungen im Hinblick auf eine Herstellung im großen Maßstab nicht berücksichtigen.

Zur Bildung von Salzen des (1R,5S)-Anhydroecgoninesters ist im Stand der Technik sehr wenig beschrieben worden. Beispielsweise seien R. C. Bick et al., Aust. J. Chem. 1979, 32, 2537-2543 und C. Grundmann et al., Liebigs Ann. Chem. 1957, 605, 24-32 genannt. In beiden Veröffentlichungen wird die Herstellung des Pikrats im experimentellen Teil kurz beschrieben. Da Pikrinsäure nicht chiral ist, kann hiermit keine Abreicherung über die Bildung von diastereomeren Salzen erreicht werden. Lediglich über die Steuerung der Ausbeute ist hier eine Abreicherung in prinzipieller Weise möglich.

In der Veröffentlichung von C. Grundmann et al. ist zusätzlich noch die Herstellung von (1R,5S)-Anhydroecgoninethylester-(2'S,3'S)-dibenzoylhydrogentartrat beschrieben. Die von C. Grundmann et al. publizierte Herstellvorschrift ist sehr aufwendig und der Lösungsmittelverbrauch extrem hoch, d.h. die Racematspaltung könnte so wie beschrieben kommerziell nicht sinnvoll umgesetzt werden.

Die Veröffentlichung von C. Grundmann et al. beschreibt ferner, dass es den Autoren bis auf die bereits erwähnte Ausnahme mit Pikrinsäure nicht gelungen ist, die Antipoden mittels L-Äpfelsäure, D-Weinsäure [(2S,3S)-Weinsäure], d-10-Camphersulfonsäure [(1S,4R)-Campher-10-sulfonsäure] und 3-Brom-d-camphersulfonsäure-7 erfolgreich zu trennen. So geht aus S. 27 hervor: "Die Spaltung des synthetischen Anhydroekgonin-äthylesters in die beiden optischen Antipoden begegnet erheblichen Schwierigkeiten. L-Äpfelsäure, D-Weinsäure, d-10-Camphersulfonsäure, 3-Brom-d-camphersulfonsäure-7 eignen sich nicht, da sie alle nur schlecht kristallisierende Salze geben."

In überraschender Weise konnte dies mit der vorliegenden Erfindung widerlegt werden.

Ferner werden in der WO 96/30371 A1 Salze von Anhydroecgoninestern zwar erwähnt, allerdings nur in Zusammenhang mit der Auftrennung von Racematen. Erfindungsgemäß dienen die Salze jedoch zur Auftrennung eines Enantiomerengemisches, bei dem das gewünschte Enantiomer stets deutlich überwiegt.

Im Stand der Technik existiert daher bislang kein effizientes, scale-up fähiges Verfahren für die Herstellung von (1R,5S)-Anhydroecgoninestern und deren Salzen.

Der vorliegenden Erfindung liegt somit die Aufgabe zugrunde, ein verbessertes Syntheseverfahren, insbesondere für den großtechnischen Maßstab, bereitzustellen, welches einen synthetischen Zugang zu den Anhydroecgoninestern bzw. deren Salze ermöglicht. Ferner soll ein wirtschaftliches, ressourcenschonendes und scale-up fähiges Verfahren mit dem Ziel einer möglichst optimalen Abreicherung des unerwünschten Enantiomers und anderer Verunreinigungen zur Verfügung gestellt werden.

### Beschreibung der Erfindung

Erfindungsgemäß wird ein für den großtechnischen Einsatz geeignetes Verfahren zur Herstellung von enantiomerenreinen (1R,5S)-Anhydroecgoninestersalzen gemäß den Lehren der Ansprüche bereitgestellt. Die Salzbildung und selektive Kristallisation von (1R,5S)-Anhydroecgoninestern mit chiralen Säuren führt mit hoher Effizienz zu einer weitgehend enantiomerenreinen Form, wobei enthaltene unerwünschte Enantiomere und andere Verunreinigungen abgereichert werden. Der Ester und dessen Salze dienen als Ausgangsmaterial für die Herstellung von Wirkstoffen.

Die vorliegende Erfindung geht in einem ersten Aspekt von (1 R,5S)-Anhydroecgoninestern der allgemeinen chemischen Formel **5** aus: 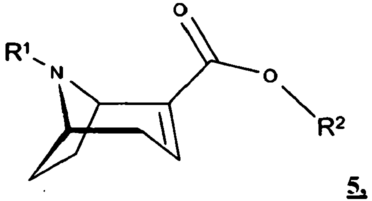 worin
R¹ Wasserstoff, einen Alkylrest, bevorzugt Methyl, Ethyl, Propyl oder Butyl, oder eine beliebige Schutzgruppe, bevorzugt Allyl, Benzyl, Methoxybenzyl, Allyloxycarbonyl, Benzyloxycarbonyl, tert.-Butyloxycarbonyl, 9-Fluorenylmethyloxycarbonyl, Acetyl, Benzoyl oder Formyl, darstellt;
R² Alkyl, Aryl, bevorzugt Phenyl oder Naphthyl, gegebenenfalls substituiert mit ein oder mehreren Substituenten, ausgewählt aus Halogen, Hydroxy, Amino, Cyano, Nitro, Trifluormethyl, Trifluormethoxy, Alkoxy, Cycloalkoxy, Alkyl, Cycloalkyl, Cycloalkylalkyl, Alkenyl und Alkinyl, darstellt; bevorzugt ist R² Methyl, Ethyl, Propyl oder Butyl.

Geeignete Schutzgruppen für R¹ können dem Stand der Technik entnommen werden, z.B. Theodora W. Green, Peter G. M. Wuts, Protective Groups in Organic Chemistry, John Wiley, 3. Auflage.

Das Herstellverfahren der neutralen Anhydroecgoninestern folgt weitgehend den Ausführungen aus dem Stand der Technik oder analog. Bezüglich nachfolgend gelisteter Schritte (1a) bis (3) wird auf S. P. Findlay, J. Org. Chem. 1957, 22, 1385-1394, dort besonders Verfahrensvariante F verwiesen, analoge Beispiele zu Schritt (4) finden sich in der Literatur und bezüglich nachfolgendem Schritt (5) wird auf die WO 2004/072071, Seite 16, Methode A Bezug genommen.

Verbindungen, die der vorliegenden Erfindung zugrunde liegen und bei denen die Substitutenten R¹ und R² von den Vorgaben aus dem vorgenannten Stand der Technik abweichen, werden analog hergestellt.

Das allgemeine Herstellverfahren zur Bereitstellung der (1R,5S)-Anhydroecgoninester wird nachfolgend im Überblick dargestellt [Verfahrensschritte (1a) bis (5)]. Im Anschluss daran wird die erfindungsgemäße Salzbildung dargestellt. Die Verfahrensschritte (3) und (4) sind dabei optional.
(1a) Umsetzen einer Verbindung der Formel **1** mit Methanol zu einer Verbindung der Formel **1'**: 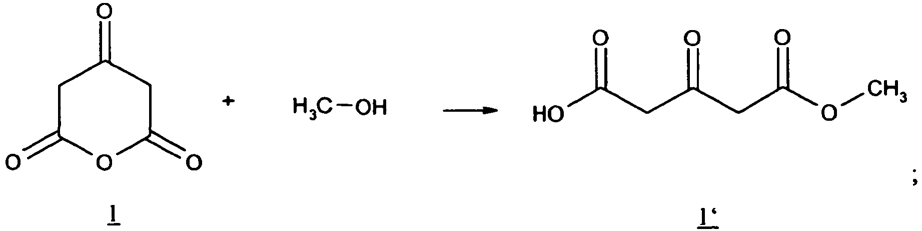
(1b) Umsetzen einer Verbindung der Formel **2** mit Wasser und Katalysator (Schwefelsäure) zu einer Verbindung der Formel **2'**: 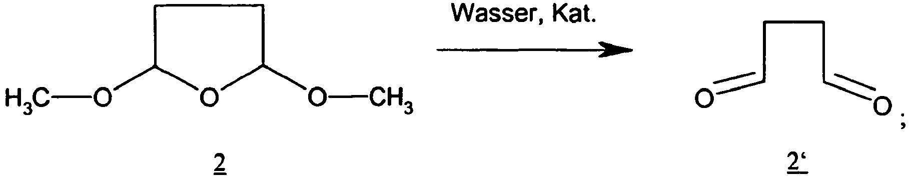
(2) Umsetzen einer Verbindung der Formel **1'** und einer Verbindung der Formel **2'** mit R¹-Amin zu einer Verbindung der Formel **3:** 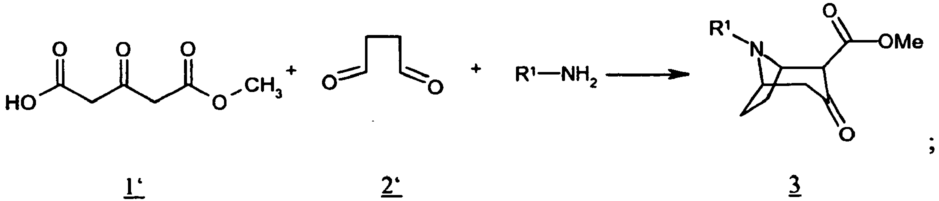 worin R¹ Wasserstoff, einen Alkylrest, bevorzugt Methyl, Ethyl, Propyl oder Butyl, oder eine beliebige Schutzgruppe, bevorzugt Allyl, Benzyl, Methoxybenzyl, Allyloxycarbonyl, Benzyloxycarbonyl, tert.-Butyloxycarbonyl, 9-Fluorenylmethyloxycarbonyl, Acetyl, Benzoyl oder Formyl, darstellt, (bevorzugt ist R¹-Amin ein Amin, ausgewählt aus der Gruppe, bestehend aus Methylamin, Ethylamin, Propylamin und Butylamin);
(3) Umsetzen der Verbindung der Formel **3** mit (2R,3R)-Weinsäure [L(+)-Weinsäure] unter Anreicherung des (1R,5S)-Enantiomer-Hydrogentartratsalzes der Verbindung der Formel **3'**: 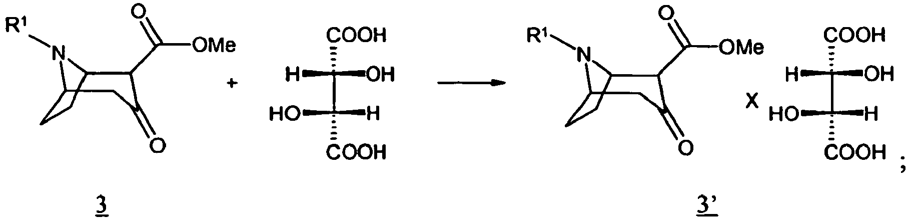
(4) Aus Verbindung **3'** kann optional in herkömmlicher Weise die weitgehend enantiomerenreine Base **4** dargestellt werden. 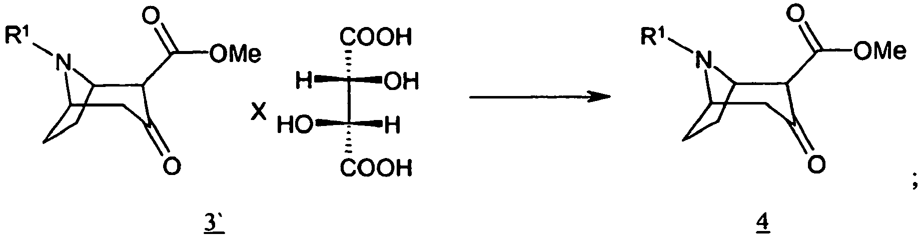
(5) Reduktion der Verbindung der Formel **4** analog WO 2004/072071 und ggf. anschließende Umesterung mit dem entsprechenden Alkoholat MOR² nach literaturbekannten Verfahren. 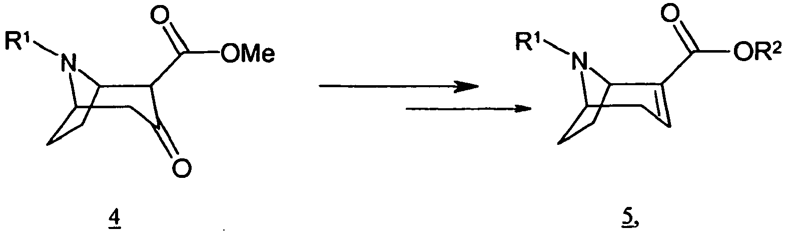 worin R² Alkyl, Aryl, bevorzugt Phenyl oder Naphthyl, gegebenenfalls substituiert mit ein oder mehreren Substituenten ausgewählt aus Halogen, Hydroxy, Amino, Cyano, Nitro, Trifluormethyl, Trifluormethoxy, Alkoxy, Cycloalkoxy, Alkyl, Cycloalkyl, Cycloalkylalkyl, Alkenyl und Alkinyl, darstellt, R² bevorzugt Methyl, Ethyl, Propyl oder Butyl
   darstellt,
   M ein Alkali- oder Erdalkalimetall, bevorzugt Kalium oder Natrium, darstellt;
(6) Umsetzen der Verbindung der Formel **5** mit einer chiralen Säure zu einem Salz der Verbindung der Formel **5'**;
(7) ggf. weitere Aufreinigung der chiralen Verbindung der Formel **5'** mittels literaturbekannter Trennverfahren und
(8) ggf. Kristallisation.

Die erfindungsgemäße Umsetzung gemäß Schritt (6) läuft nach einer der nachfolgenden Reaktionsgleichungen ab: 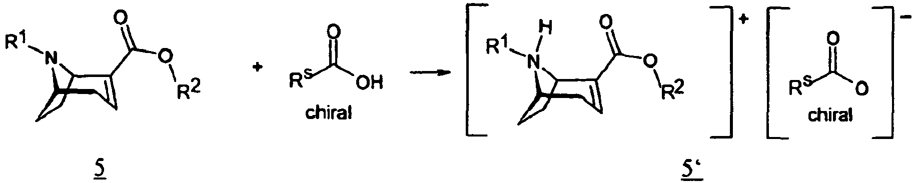 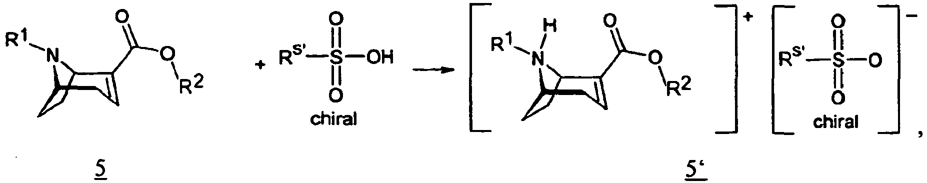 wobei R¹ und R² wie oben definiert sind und R^{s} und R^{s'} die Säurereste der verwendeten chiralen Säuren darstellen.

Die Erfindung betrifft in einem zweiten Aspekt auch Salze der Verbindung der Formel **5'**: 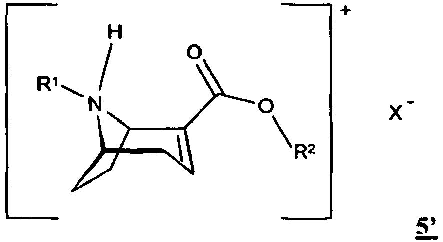 mit X⁻:

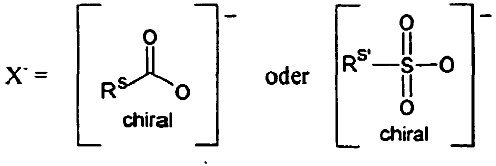

Ein weiterer Aspekt der vorliegenden Erfindung ist ein Verfahren gemäß den Schritten (1), (2), (5) und (6), ggf. unter Einbeziehung der Schritte (3) und (4).

### Detaillierte Beschreibung der Erfindung

### Begriffsdefinitionen

Der Begriff "Aryl" bzw. "Aryl-Rest" bedeutet einen 6- bis 10-gliedrigen aromatischen Carbocyclus und umfasst beispielsweise Phenyl und Naphthyl. Andere Begriffe, welche als Bestandteil den Begriff "Aryl" enthalten, weisen dieselbe Bedeutung für den Aryl-Bestandteil auf. Beispiele für diese Komponenten sind: Arylalkyl, Aryloxy oder Arylthio.

Als "Alkyl" bzw. "Alkylgruppen" sowie Alkylgruppen, welche Bestandteil anderer Reste sind, werden verzweigte und unverzweigte Alkylgruppen mit 1 bis 6 Kohlenstoffatomen bezeichnet. Beispielsweise werden genannt: Methyl, Ethyl, Propyl, Butyl, Pentyl, Hexyl. Sofern nicht anders angegeben, sind von den vorstehend genannten Bezeichnungen Propyl, Butyl, Pentyl und Hexyl sämtliche der möglichen isomeren Formen umfaßt. Beispielsweise umfaßt die Bezeichnung Propyl die beiden isomeren Reste n-Propyl und iso-Propyl, die Bezeichnung Butyl die isomeren Reste n-Butyl, iso-Butyl, sek. Butyl und tert.-Butyl.

Als "Alkoxy" oder "Alkyloxygruppen", werden verzweigte und unverzweigte Alkylgruppen mit 1 bis 6 Kohlenstoffatomen bezeichnet, die über ein Sauerstoffatom verknüpft sind. Beispielsweise werden genannt: Methoxy, Ethoxy, Propoxy, Butoxy, Pentoxy, Hexoxy. Sofern nicht anders angegeben, sind von den vorstehend genannten Bezeichnungen sämtliche der möglichen isomeren Formen umfaßt.

Alkenylgruppen bedeuten verzweigte und unverzweigte Alkenylgruppen mit 2 bis 6 Kohlenstoffatomen, bevorzugt 2 bis 4 Kohlenstoffatomen, die mindestens eine Doppelbindung aufweisen, wie zum Beispiel die oben erwähnten Alkylgruppen, vorausgesetzt, sie haben mindestens eine Doppelbindung im Molekül, beispielsweise Vinyl, Propenyl, Isopropenyl, Butenyl, Pentenyl und Hexenyl.

Alkenylengruppen sind verzweigte und unverzweigte Alkenylbrücken mit 2 bis 6 Kohlenstoffatomen, bevorzugt 2 bis 4 Kohlenstoffatomen mit mindestens einer Doppelbindung im Molekül, z.B. die oben erwähnten Alkylengruppen, vorausgesetzt, sie haben mindestens eine Doppelbindung, wie beispielsweise Vinylen, Propenylen, Isopropenylen, Butenylen, Pentenylen und Hexenylen.

Wenn nicht anders spezifiziert, sollen die oben erwähnten Alkenyl- und Alkenylengruppen so verstanden werden, dass sie gegebenenfalls existierende Stereoisomere umfassen. Demgemäß soll beispielsweise die Definition 2-Butenyl so verstanden werden, dass sie 2-(Z)-Butenyl und 2-(E)-Butenyl etc. umfasst.

Der Begriff Alkinylgruppen bezieht sich auf Alkinylgruppen mit 2 bis 6, bevorzugt 2 bis 4 Kohlenstoffatomen, vorausgesetzt, sie weisen mindestens eine Dreifachbindung im Molekül auf, z.B. Ethinyl, Propargyl, Butinyl, Pentinyl und Hexinyl.

Halogen steht für Fluor, Chlor, Brom oder Iod, bevorzugt Chlor oder Brom.

Als "carbocyclischer Ring" oder "Cycloalkylgruppen" werden Cycloalkylreste mit 3 bis 6 Kohlenstoffatomen, beispielsweise Cyclopropyl, Cyclobutyl, Cyclopentyl oder Cyclohexyl bezeichnet.

Der Begriff "Stickstoff" sowie das entsprechende Elementsymbol, umfasst jegliche oxidierte Form dieses und auch quaternäre Formen eines basischen Stickstoffatoms sollen umfasst sein.

In spezifischen Ausführungsformen der Erfindung bedeutet der Begriff "etwa" innerhalb 20%, bevorzugt innerhalb 10% und bevorzugter innerhalb 5% eines vorgegebenen Wertes oder Bereichs. Ein angegebener Wertebereich umfasst und offenbart sämtliche darin enthaltenen Werte und Werteintervalle.

Sollte eine chemische Formel mit einer chemischen Bezeichnung in Widerspruch stehen und der Fachmann anhand seines Wissens und seiner Kenntnisse nicht ohne weiteres in der Lage sein, diesen Widerspruch zu lösen, so soll im Zweifel die Formel ausschlaggebend sein.

### Bevorzugte Ausführungformen

Nachfolgend wird das erfindungsgemäße Verfahren im Einzelnen beschrieben. Die Herstellung des für die erfindungsgemäße Salzbildung notwendigen Ausgangsprodukts **5** wird im nachfolgenden Reaktionsschema 1 dargestellt:

### Reaktionsschema 1: Totalsynthese von Anhydroecgoninestern:

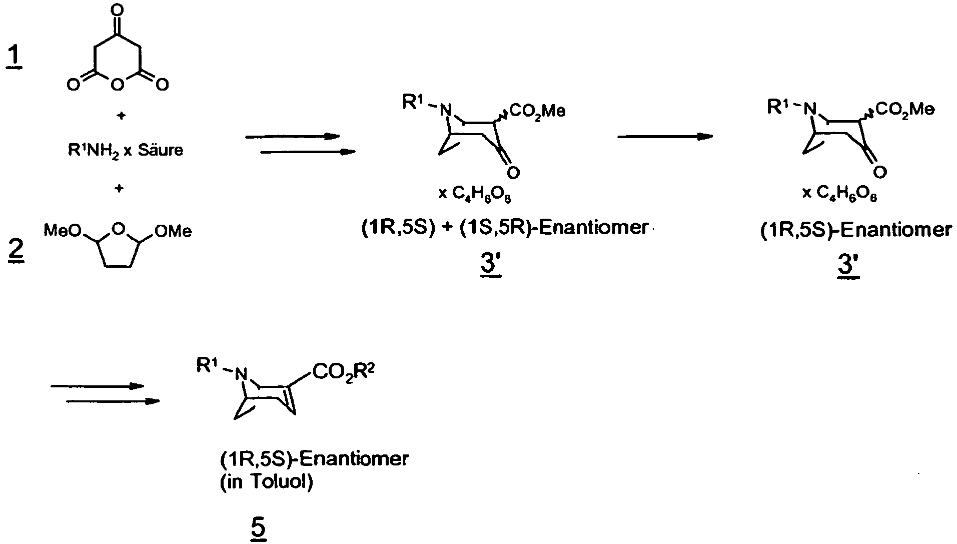

Me steht für Methyl.

Wie bereits ausgeführt folgt die Synthese von **5** dem Stand der Technik, insbesondere S. P. Findlay, J. Org. Chem. 1957, 22, 1385-1394, dort besonders Verfahrensvariante F und WO 2004/072071, Seite 16, Methode A.

Die erfindungsgemäße Umsetzung der Verbindung der Formel **5** zum Salz kann sich unmittelbar an Schritt (5) anschließen. Hierzu wird in Schritt (6) eine Verbindung der Formel **5** mit einer chiralen Säure zu einem Salz der Verbindung der Formel **5'** gemäß der nachfolgenden Reaktionsgleichungen umgesetzt:

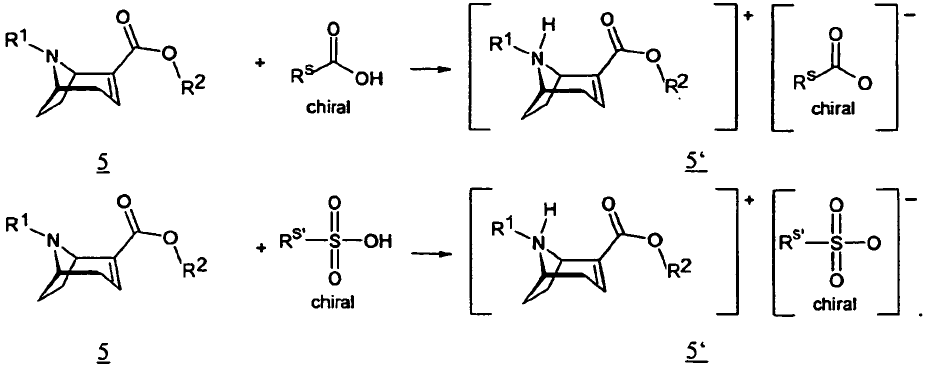

Die Kristallisation von Verbindungen der Formel **5** (1R,5S)-Anhydroecgoninester mit optisch aktiven Säuren ist nicht trivial. Es wurden zahlreiche chirale Säuren unter verschiedenen Bedingungen bezüglich ihrer Kristallisationsfähigkeit mit der Verbindung der Formel **5** untersucht. Für die Salzbildung liegt die Verbindung der Formel **5** zunächst in einem Lösungsmittel gelöst vor, bevorzugt in konzentrierter Form ("Konzentrat"). Unter den Begriffen "in konzentrierter Form" oder "Konzentrat" wird dabei eine wenigstens 20 Gew.%ige, bevorzugt wenigstens 30 Gew.%ige, stärker bevorzugt wenigstens 40 Gew.%ige, stärker bevorzugt wenigstens 50 Gew.%ige und noch stärker bevorzugt wenigstens 60 Gew.%ige Lösung verstanden. Als Lösungsmittel dienen organische Lösungsmittel mit einem Siedepunkt unterhalb von 150°C (bei p = 1 bar), bevorzugt sind Toluol, Xylol (alle Isomere), Halogenbenzole, aliphatische Kohlenwasserstoffe (C₅ bis C₈), halogenhaltige, aliphatische Kohlenwasserstoffe (C₁ bis C₆), aliphatische Ether (C₄ bis C₈), Ester der Ameisensäure (C₂ bis C₇), Ester der Essigsäure (C₃ bis C₇) oder Nitrile (C₂ bis C₅).

Am stärksten bevorzugt sind aromatische Kohlenwasserstoffe. Genannt werden Toluol und XyloL Am meisten bevorzugt ist Toluol. Sollte sich die Verbindung der Formel **5** zunächst nicht in dem Kristallisationslösungsmittel lösen, kann sie zuvor in einem geeigneten anderen Lösungsmittel, beispielsweise einem Alkohol wie Methanol oder Ethanol gelöst werden, um so das gewünschte Konzentrat zugänglich zu machen.

Nachfolgend sind beispielhaft einige Bedingungen zusammengefasst, welche besonders bevorzugt sind, um die salzbildung zu ermöglichen:

Die Salzbildung findet bevorzugt in toluolischer Lösung statt. Dafür wird die Verbindung **5** in Toluol gelöst, ggf. kann die Verbindung zunächst in einem anderen Lösungsmittel gelöst vorliegen, beispielsweise dem Lösungsmittel aus dem vorgeschalteten Reaktionsschritt. Bevorzugt liegt die Verbindung **5** zunächst in einem Alkohol gelöst vor, beispielsweise Methanol oder Ethanol. Vorzugsweise wird die chirale Säure in einem Lösungsmittel vorgelegt und hierzu das toluolische Konzentrat der Verbindung der Formel **5**, bevorzugt unter Rühren, zugegeben. Durch die Salzbildung gelingt es etwaig vorhandene Enantiomerengemische aufzutrennen, so dass das Produkt, das (1R,5S)-Enantiomer, in einer Enantiomerenreinheit über etwa 95%, bevorzugter über etwa 98%, insbesondere über etwa 99%, ganz besonders bevorzugt über etwa 99,9% abgetrennt werden kann.

Beispiele von chiralen Säuren, die verwendet werden können, sind: (1S,4R)-Campher-10-sulfonsäure, (1R,4S)-Campher-10-sulfonsäure, (2R,3R)-Di-p-toluoylweinsäure, (2S,3S)-Di-p-toluoylweinsäure, (2R,3R)-Weinsäure, (2S,3S)-Weinsäure, (2R,3R)-Dibenzoylweinsäure und (2S,3S)-Dibenzoylweinsäure.

In überraschender Weise konnte entgegen der Auffassung im Stand der Technik (siehe supra, C. Grundmann et al.) ein Salz mit (1S,4R)-Campher-10-sulfonsäure (= d-10-Camphersulfonsäure) erhalten werden. Das Campher-10-sulfonat kristallisiert in guter Qualität, wobei das unerwünschte (1S,5R)-Enantiomer sehr gut abgereichert werden kann.

Die Ausbeute kann entsprechend optimiert werden, beispielsweise durch gezielte Verlangsamung des Kristallisationsprozesses, Arbeiten bei niedrigeren Temperaturen am Ende des Kristallisationsprozesses oder ähnliches.

Im einzelnen wird zur Herstellung der Salze der Verbindung der Formel **5'** zunächst die chirale Säure vorzugsweise in einem Lösungsmittel, bevorzugt Aceton, bei erhöhter Temperatur, beispielsweise 35 bis 60°C, bevorzugt etwa 40 bis 50°C, in einzelnen Fällen unter Erhitzen bis auf Rückflusstemperatur des Lösungsmittels, gelöst. Als Lösungsmittel können anstelle von Aceton auch Alkohole (C₁ bis C₅), Nitrile (C₂ bis C₃) und Ketone (C₃ bis C₆) eingesetzt werden. Alle polaren und mittelpolaren protischen oder aprotischen Lösungsmittel mit und ohne Zusatz variierender Mengen von H₂O sind generell möglich. Dies hängt von der jeweils verwendeten Säure ab. In Einzelfällen kann es sinnvoll sein, die erhaltene Lösung heiß zu filtrieren.

Anschließend wird die Verbindung der Formel **5**, gegebenenfalls gelöst in einem der oben genannten Lösungsmittel, bevorzugt unter Rühren zur Lösung der chiralen Säure zugegeben. Als Edukt kann die Verbindung der Formel **5** mit variablem Lösungsmittelgehalt eingesetzt werden, wobei vorteilhafterweise das Toluol-Konzentrat, das in Schritt (5) erhalten wurde, gegebenenfalls unter Zugabe eines weiteren Lösungsmittels, unmittelbar eingesetzt wird. Die Verbindung der Formel **5** kann als Enantiomeren-Gemisch zum Einsatz kommen, das neben dem gewünschten Enantiomer [(1R,5S)-Enantiomer] zusätzlich Anteile des unerwünschten Enatiomeren [(1S,5R)-Enantiomer] enthält. Beispielsweise kann die Verbindung der Formel **5** mit einer Enantiomerenreinheit ≥ 80%, bevorzugt ≥ 90% des gewünschten Enantiomers [(1R,5S)-Anhydroecgoninester] und entsprechenden Anteilen des unerwünschten Enantiomeren zum Einsatz kommen.

Besonders bevorzugt befindet sich die Lösung der chiralen Säure noch bei erhöhter Temperatur, besonders bevorzugt bei oder in der Nähe der Temperatur, bei der die chirale Säure gelöst wurde, während die Verbindung der Formel **5** zugesetzt wird. Dies bedeutet, dass die Lösungstemperatur für die chirale Säure bei der Zugabe der Verbindung der Formel **5** möglichst nur um etwa 20°C, vorzugsweise etwa 10°C, noch bevorzugter etwa 5°C unterschritten wird.

Die erhaltene Lösung kann optional nach Erhitzen bis auf Rückflusstemperatur des Lösungsmittels abgekühlt werden, wonach, gegebenenfalls nach Animpfen mit einer kleinen Menge an Impfkristallen und/oder Anreiben, das gewünschte Enantiomer als Salz mit einer Enantiomerenreinheit ≥ 98%, bevorzugt ≥ 99%, besonders bevorzugt ≥ 99,9%, ausfällt. Die Endtemperatur des (1R,5S)-Enantiomers beim Abscheiden liegt besonders bevorzugt zwischen -15 und 35°C, insbesondere 5 bis 35°C. Das Abscheiden des Salzes aus dem Lösungsmittel wird besonders bevorzugt in Verdünnungen (Σ m_{Edukte} : Σ V_{Lösungsmittel}) von 1:10 bis 2:1 durchgeführt.

Nach Abtrennung in geeigneter Weise kann durch entsprechende Aufarbeitung, wie Waschen mit Lösungsmittel und weitere Reinigung, beispielsweise chromatographische Verfahren und dergleichen, eine Enantiomerenreinheit von ≥ 99,9% des (1R,5S)-Enantiomers in Form des Salzes erhalten werden.

Anders als die Vorschriften aus dem Stand der Technik (C. Grundmann et al.), die sich nicht in den technischen Maßstab übertragen lassen, ist dies beim erfindungsgemäßen Verfahren möglich.

Das toluolische Konzentrat kann ohne Vorbereitungs- bzw. Aufarbeitungsschritt, wie zum Beispiel Entfernen des Toluols im Vakuum, direkt eingesetzt werden oder es kann ebenfalls ein Ausgangsmaterial aus einer kommerziellen Quelle auch in Form eines Enantiomerengemischs zum Einsatz kommen, wobei durch Abreicherung eines der beiden Enantiomeren, das gewünschte Enantiomer als Salz mit einer Enantiomerenreinheit ≥ 98%, bevorzugt ≥ 99%, besonders bevorzugt ≥ 99,9% erhalten wird.

Gegenstand der Erfindung sind auch die Salze der Verbindung der Formel **5'** mit einer chiralen Säure. Die chirale Säure wird vorzugsweise ausgewählt aus: (1S,4R)-Campher-10-sulfonsäure, (1R,4S)-Campher-10-sulfonsäure, (2R,3R)-Di-p-toluoylweinsäure, (2S,3S)-Di-p-toluoylweinsäure, (2R,3R)-Weinsäure, (2S,3S)-Weinsäure, (2R,3R)-Dibenzoylweinsäure und (2S,3S)-Dibenzoylweinsäure. Nachfolgend werden einige Beispiele chiraler Säuren dargestellt:

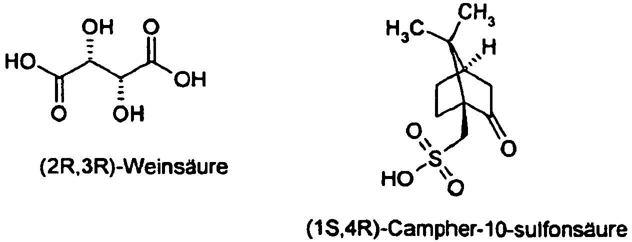

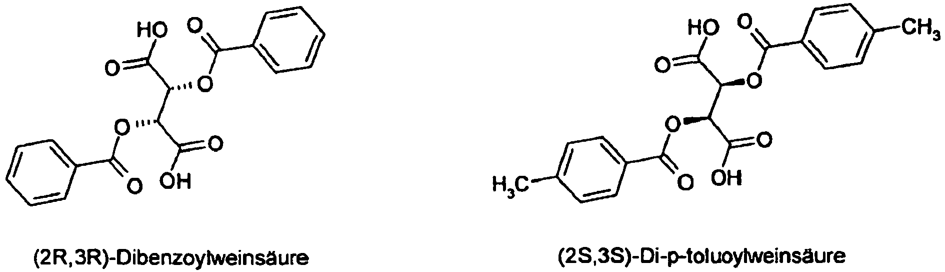

Die chiralen Säuren sind selbstverständlich nicht auf die genannten chiralen Säuren beschränkt. Dem Fachmann sind weitere chirale Säuren bekannt, die ebenfalls zur Herstellung von Salzen der Verbindung der Formel **5'** eingesetzt werden können.

Erfindungsgemäß ganz besonders bevorzugt sind Salze der folgenden Ester: (1R,5S)-Anhydroecgoninethylester, (1R,5S)-Anhydroecgoninmethylester, (1R,5S)-Anhydroecgoninpropylester und (1R,5S)-Anhydroecgoninbutylester.

Nachfolgend ist ein erfindungsgemäß besonders bevorzugter (1R,5S)-Anhydroecgoninethylester dargestellt:

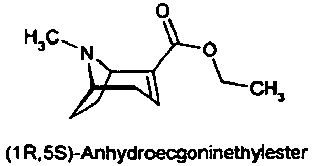

Insbesondere bevorzugt stellt das Salz der Verbindung der Formel **5'** eine der nachfolgenden Verbindungen dar:
(1R,5S)-Anhydroecgoninethylester-(1'S,4'R)-campher-10-sulfonat;
(1R,5S)-Anhydroecgoninethylester-(1'R,4'S)-campher-10-sulfonat;
(1R,5S)-Anhydroecgoninethylester-(2'S,3'S)-di-p-toluoylhydrogentartrat;
(1R,5S)-Anhydroecgoninethylester-(2'R,3'R)-di-p-toluoylhydrogentartrat;
(1R,5S)-Anhydroecgoninethylester-(2'R,3'R)-hydrogentartrat-monohydrat;
(1R,5S)-Anhydroecgoninethylester-(2'S,3'S)-hydrogentartrat-monohydrat;
(1R,5S)-Anhydroecgoninethylester-(2'R,3'R)-dibenzoylhydrogentartrat;
(1R,5S)-Anhydroecgoninethylester-(2'S,3'S)-dibenzoylhydrogentartrat.

Überraschenderweise wurde von (2R,3R)-Weinsäure mit (1R,5S)-Anhydroecgoninmethylester in Gegenwart von Wasser ein gut kristallisierbares Monohydrat hergestellt. Es handelt sich um das erste isolierte Monohydrat eines Anhydroecgoninethylester-Salzes.

Die Verwendung von Salzen der Verbindung der Formel **5'** gegenüber der Verwendung des toluolischen Konzentrats der Verbindung **5** hat eine Reihe an Vorteilen:

So lässt sich beim toluolischen Konzentrat der Gehalt an Stickstoff-Basen nicht ohne größeren analytischen oder betrieblichen Aufwand konstant einstellen. Nach Versuchen schwankt dieser Wert in hergestellten Chargen zwischen 65 und 95 Gew. %, so dass die Weiterverarbeitung des toluolischen Konzentrats Schwierigkeiten aufwerfen kann.

Das toluolische Konzentrat lässt sich im Gegensatz zum Salz nicht weiter reinigen. Das toluolische Konzentrat kann zwar prinzipiell im Vakuum destilliert werden, jedoch zeigte ein Laborversuch, dass trotz großzügigen Schneidens der Hauptfraktion nur eine geringe Verbesserung im Hinblick auf die chromatographische Reinheit erhalten werden konnte. Lediglich das Lösungsmittel konnte abgetrennt werden, aber es konnte keine signifikante Reinigung erreicht werden. Demgegenüber kann das Salz beispielsweise durch Umkristallisieren gereinigt werden.

Ferner ist die Stabilität der Verbindung der Formel **5** in der Regel in Lösung geringer als in Feststoffform, d.h. als auskristallisiertes Salz. Dies hat Auswirkungen auf die Aufbewahrungsdauer, Haltbarkeit und Lagerung, die für längere Zeit in Lösungsform nicht möglich ist.

Weiterhin ist der Transport und die Lagerung von Feststoffen in der Regel sicherer und einfacher möglich als der Transport von Flüssigkeiten, die größeren Platz erfordern und in entsprechend flüssigkeitsdichten Behältern transportiert werden müssen.

Auch vom Erscheinungsbild im Hinblick auf das Aussehen hat der Feststoff Vorteile gegenüber der Lösung des Produkts. So fällt das toluolische Konzentrat als orange-braune Lösung an, die Salze hingegen werden in der Regel in Form eines weißen Feststoffes isoliert.

Zudem können Salze der Anhydroecgoninester per se genauer und einfacher analytisch charakterisiert werden als Lösungen bzw. Konzentrate der Anhydroecgoninester.

Gegenüber dem Stand der Technik, insbesondere der Vorschrift von C. Grundmann et al. wird eine deutlich höhere Ausbeute erhalten. Das erfindungsgemäße Verfahren ist ressourcenschonender, da die Lösungsmittelmenge um ein Vielfaches reduziert werden kann und das Verfahren damit preiswerter ist.

Das erfindungsgemäße totalsynthetische Herstellverfahren vermeidet den Einsatz von Kokainhydrochlorid als Edukt; dennoch wird in effizienter Weise das Produkt mit sehr hohem ee-Wert erhalten, so dass die Bereitstellung auch größerer Mengen an reinen (1R,5S)-Anhydroecgoninester und/oder dessen Salze als Ausgangsmaterialien für die Herstellung von Wirkstoffen zur Verfügung gestellt werden können.

### Zusammenfassend stellt sich die Erfindung wir folgt dar:

Ein erster Aspekt 1 der vorliegenden Erfindung betrifft ein Verfahren zur Herstellung von chiralen Salzen von (1R,5S)-Anhydroecgoninestern der allgemeinen chemischen Formel **5,** welche bevorzugt in enantiomeren-angereicherter Form anfallen: 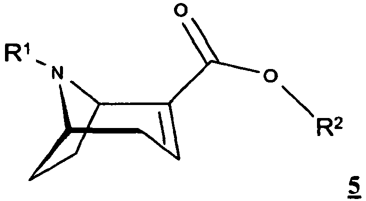 worin
R¹ Wasserstoff, einen Alkylrest, bevorzugt Methyl, Ethyl, Propyl oder Butyl, oder eine beliebige Schutzgruppe, bevorzugt Allyl, Benzyl, Methoxybenzyl, Allyloxycarbonyl, Benzyloxycarbonyl, tert.-Butyloxycarbonyl, 9-Fluorenylmethyloxycarbonyl, Acetyl, Benzoyl oder Formyl, darstellt;
R² Alkyl, Aryl, bevorzugt Phenyl oder Naphthyl, gegebenenfalls substituiert mit ein oder mehreren Substituenten ausgewählt aus Halogen, Hydroxy, Amino, Cyano, Nitro, Trifluormethyl, Trifluormethoxy, Alkoxy, Cycloalkoxy, Alkyl, Cycloalkyl, Cycloalkylalkyl, Alkenyl und Alkinyl, darstellt; bevorzugt ist R² Methyl, Ethyl, Propyl oder Butyl
mit den Schritten
(1a) Umsetzen einer Verbindung der Formel **1** mit Methanol und ggf. in Methanol als Lösungsmittel zu einer Verbindung der Formel **1'**: 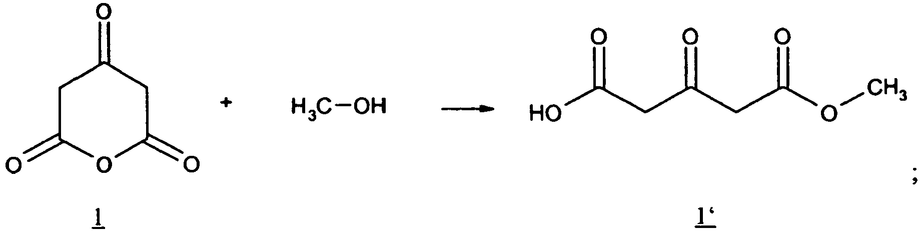
(1b) Umsetzen einer Verbindung der Formel **2** mit Wasser und Katalysator zu einer Verbindung der Formel **2'** 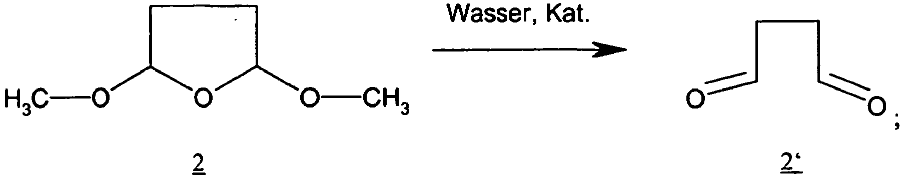
(2) Umsetzen einer Verbindung der Formel **1'** und einer Verbindung der Forme **2'** mit einer R¹-Amin-Lösung zu einer Verbindung der Formel **3**: 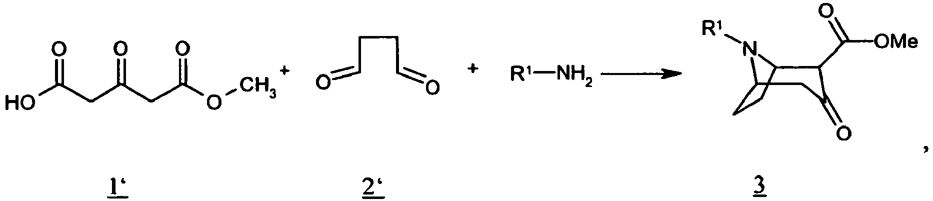 worin R¹ Wasserstoff, einen Alkylrest, bevorzugt Methyl, Ethyl, Propyl oder Butyl, oder eine beliebige Schutzgruppe, bevorzugt Allyl, Benzyl, Methoxybenzyl, Allyloxycarbonyl, Benzyloxycarbonyl, tert.-Butyloxycarbonyl, 9-Fluorenylmethyloxycarbonyl, Acetyl, Benzoyl oder Formyl, darstellt;
(3) optional Umsetzen der Verbindung der Formel **3** mit (2R,3R)-Weinsäure [L(+)-Weinsäure] unter Anreicherung des (R)-Enantiomer-Hydrogentartratsalzes der Verbindung der Formel **3'**: 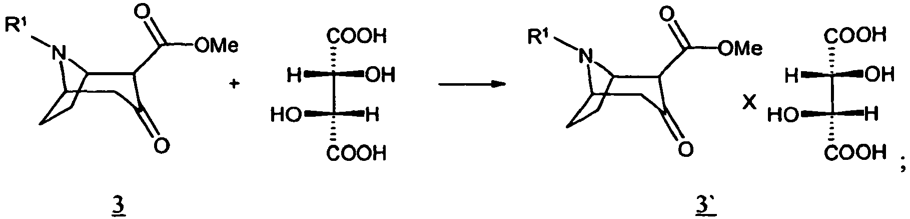
(4) optional Freisetzung der Verbindung **4** im Basischen 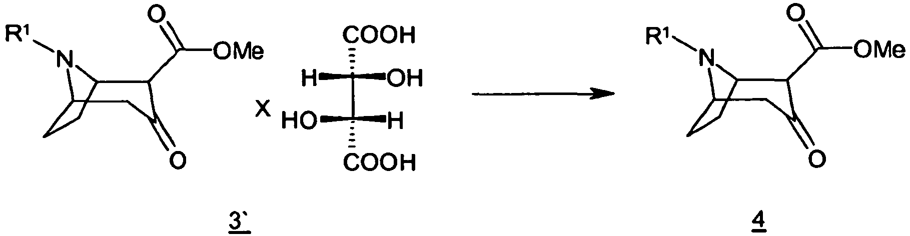
(5) Reduktion des erhaltenen Produkts und ggf. weitere Umesterung mit einem Alkoholat der Formel MOR² zu einer Verbindung der Formel **5**: 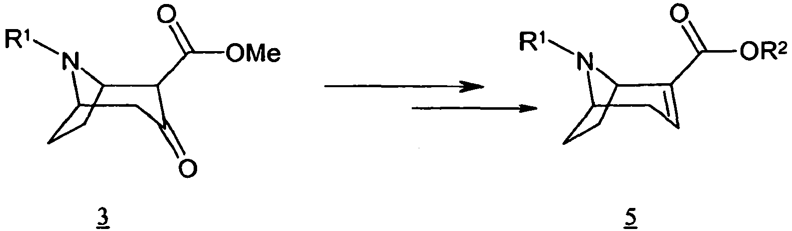 worin R² Alkyl, Aryl, bevorzugt Phenyl oder Naphthyl, gegebenenfalls substituiert mit ein oder mehreren Substituenten ausgewählt aus Halogen, Hydroxy, Amino, Cyano, Nitro, Trifluormethyl, Trifluormethoxy, Alkoxy, Cycloalkoxy, Alkyl, Cycloalkyl, Cycloalkylalkyl, Alkenyl und Alkinyl, darstellt, R² bevorzugt Methyl, Ethyl, Propyl oder
   Butyl
   darstellt,
   M ein Alkali- oder Erdalkalimetall, bevorzugt Kalium oder Natrium, darstellt; und
(6) Umsetzen der Verbindung der Formel **5** mit einer chiralen Säure zu einem Salz der Verbindung der Formel **5'**: 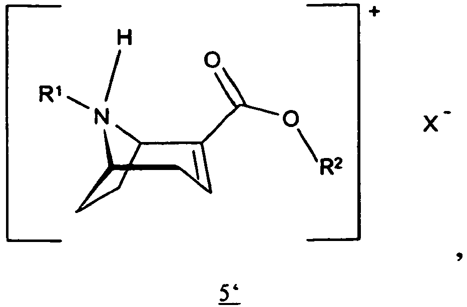 wobei X- das Anion einer chiralen Säure darstellt;
(7) ggf. Aufreinigung der chiralen Verbindung der Formel **5'** und
(8) ggf. Kristallisation.

In dem Verfahren nach Aspekt 1 der Erfindung kann am Ende der Aufarbeitung in Schritt (5) durch Zugabe von Toluol eine toluolische konzentrierte Lösung der Verbindung der Formel **5** hergestellt werden (Variante 2).

In dem Verfahren nach Aspekt 1 der Erfindung kann im Schritt (6) die Verbindung der Formel **5** in Toluol als Konzentrat gelöst zu wenigstens 20 Gew.% vorliegen, bevorzugt zu wenigstens 40 Gew.%, besonders bevorzugt zu wenigstens 60 Gew.% (Variante 3).

In dem Verfahren nach Aspekt 1 der Erfindung kann in Schritt (6) die chirale Säure in einem Lösungsmittel vorgelegt werden und hierzu die toluolische konzentrierte Lösung der Verbindung der Formel **5** zugegeben werden (Variante 4).

In dem Verfahren nach Aspekt 1 der Erfindung und in dessen Varianten 2, 3 und 4, kann die chirale Säure ausgewählt sein aus: (1S,4R)-Campher-10-sulfonsäure, (1R,4S)-Campher-10-sulfonsäure, (2R,3R)-Di-p-toluoylweinsäure, (2S,3S)-Di-p-toluoylweinsäure, (2R,3R)-Weinsäure, (2S,3S)-Weinsäure, (2R,3R)-Dibenzoylweinsäure und (2S,3S)-Dibenzoylweinsäure (Variante 5).

In dem Verfahren nach Aspekt 1 der Erfindung kann das Lösungsmittel in Schritt (6) ausgewählt werden aus polaren bzw. mittelpolaren protischen oder aprotischen Lösungsmitteln, bevorzugt Aceton, C₁- bis C₅-Alkoholen, C₂- bis C₃-Nitrilen, C₃- bis C₆-Ketonen, mit oder ohne Zusatz von Wasser (Variante 6).

In dem Verfahren nach Aspekt 1 der Erfindung kann die chirale Säure im Lösungsmittel in Schritt (6) unter Erhitzen auf eine Temperatur im Bereich von etwa 35°C bis etwa zur Rückflusstemperatur des verwendeten Lösungsmittels gelöst werden (Variante 7).

In dem Verfahren nach Aspekt 1 der Erfindung kann die toluolische konzentrierte Lösung der Verbindung der Formel **5** zu der Lösung der chiralen Säure in Schritt (6) bei oder in der Nähe der Lösungstemperatur der chiralen Säure zugesetzt werden (Variante 8).

In dem Verfahren nach Aspekt 1 der Erfindung und auch in dessen Varianten 7 oder 8 kann in Schritt (6) nach Zugabe der toluolischen konzentrierten Lösung und gegebenenfalls Erhitzen bis auf Rückflusstemperatur des Lösungsmittels, abgekühlt werden (Variante 9).

In dem Verfahren nach Aspekt 1 der Erfindung kann die Verbindung der Formel **5'** bei einer Endtemperatur zwischen -15 und 35°C, bevorzugt, 5 bis 35°C ausgefällt werden (Variante 10).

In dem Verfahren nach Aspekt 1 der Erfindung kann das Ausfällen durch Animpfen mit einer kleinen Menge an Impfkristallen und/oder Anreiben unterstützt werden (Variante 11).

In dem Verfahren nach Aspekt 1 der Erfindung kann in Schritt (6), die Verbindung der Formel **5'** in Form eines Enantiomeren in einer Enantiomerenreinheit von über etwa 95%, bevorzugt über etwa 96%, besonders bevorzugt über etwa 98%, insbesondere über etwa 99%, ganz besonders bevorzugt über etwa 99,9% ausgefällt werden (Variante 12).

Ein zweiter Aspekt der Erfindung betrifft ein Verfahren zur Herstellung von Salzen der Verbindung der Formel **5**: 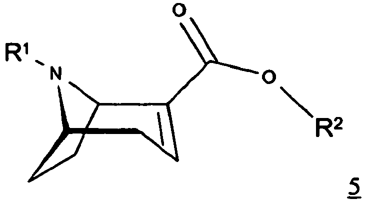 wobei die Verbindung der Formel 5 mit einer chiralen Säure zu einem Salz der Verbindung der Formel **5'** umgesetzt wird: 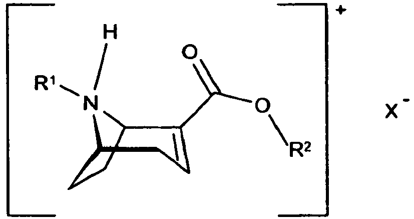 wobei R¹ und R² wie in Anspruch 1 definiert sind, und
X⁻ das Anion einer chiralen Säure darstellt. Wobei die Verbindung nach Formel **5** bevorzugt bezüglich eines der möglichen Enantiomere angereichert vorliegt.

In dem Verfahren nach Aspekt 2 der Erfindung kann die chirale Säure, ausgewählt werden aus der Gruppe, bestehend aus: (1S,4R)-Campher-10-sulfonsäure, (1R,4S)-Campher-10-sulfonsäure, (2R,3R)-Di-p-toluoylweinsäure, (2S,3S)-Di-p-toluoylweinsäure, (2R,3R)-Weinsäure, (2S,3S)-Weinsäure, (2R,3R)-Dibenzoylweinsäure und (2S,3S)-Dibenzoylweinsäure, stärker bevorzugt (1S,4R)-Campher-10-sulfonsäure, (1R,4S)-Campher-10-sulfonsäure, (2R,3R)-Di-p-toluoylweinsäure, (2S,3S)-Di-p-toluoylweinsäure, (2R,3R)-Weinsäure, (2S,3S)-Weinsäure und (2R,3R)-Dibenzoylweinsäure (Variante 2.1).

In dem Verfahren nach Aspekt 2 der Erfindung und ebenfalls der Variante 2.1 kann die chirale Säure in einem Lösungsmittel bzw. Lösungsmittelgemisch vorgelegt und hierzu eine toluolische konzentrierte Lösung der Verbindung der Formel **5** zugegeben werden, in welcher die Verbindung der Formel **5** bevorzugt zu wenigstens 60 Gew.% vorliegt (Variante 2.2).

In dem Verfahren nach Aspekt 2 der Erfindung und auch in den Varianten 2.1 und 2.2 kann das Lösungsmittel ausgewählt werden aus polaren protischen oder aprotischen Lösungsmitteln, bevorzugt Aceton, C₁- bis Cs-Alkoholen, C₂- bis C₃-Nitrilen, C₃- bis C₆-Ketonen, mit oder ohne Zusatz von Wasser (Variante 2.3).

In dem Verfahren nach Aspekt 2 der Erfindung und auch in den Varianten 2.1, 2.2 und 2.3 kann die chirale Säure im Lösungsmittel unter Erhitzen auf eine Temperatur im Bereich von etwa 35°C bis etwa zur Rückflusstemperatur des verwendeten Lösungsmittels gelöst werden (Variante 2.4).

In dem Verfahren nach Aspekt 2 der Erfindung und auch in den Varianten 2.1, 2.2, 2.3 und 2.4 kann die toluolische konzentrierte Lösung der Verbindung der Formel **5** zur Lösung der chiralen Säure bei oder in der Nähe der Lösungstemperatur der chiralen Säure zugesetzt werden (Variante 2.5).

In dem Verfahren nach Aspekt 2 der Erfindung und auch in den Varianten 2.1 bis 2.5 kann nach Zugabe der toluolischen konzentrierten Lösung und gegebenenfalls Erhitzen bis auf Rückflusstemperatur des Lösungsmittels, abgekühlt werden (Variante 2.6).

In dem Verfahren nach Aspekt 2 der Erfindung und auch in den Varianten 2.1 bis 2.6 kann die Verbindung der Formel **5'** bei einer Endtemperatur zwischen -15 und 35°C, bevorzugt, 5 bis 35°C ausgefällt werden (Variante 2.7).

In dem Verfahren nach Aspekt 2 der Erfindung und auch in den Varianten 2.1 bis 2.7 kann das Ausfällen durch Animpfen mit einer kleinen Menge an Impfkristallen und/oder Anreiben unterstützt werden (Variante 2.8).

In dem Verfahren nach Aspekt 2 der Erfindung und auch in den Varianten 2.1 bis 2.8 kann die Verbindung der Formel **5'** in Form eines Enantiomeren in einer Enantiomerenreinheit von über etwa 95%, bevorzugt über etwa 96%, besonders bevorzugt über etwa 98%, insbesondere über etwa 99%, ganz besonders bevorzugt über etwa 99,9% ausgefällt werden (Variante 2.9).

Ein dritter Aspekt der Erfindung betrifft ein enantimerenreines Salz der Verbindung der Formel **5** mit einer chiralen Säure.

Ein vierter Aspekt der Erfindung betrifft ein chirales, bevorzugt enantiomerenreines Salz der Verbindung der Formel **5** mit einer chiralen Säure, welches kristallin ist.

Das Salz nach einem der Aspekte 3 oder 4 der Erfindung schließt bevorzugt (1R,5S)-Anhydroecgoninethylester-(2'S,3'S)-dibenzoylhydrogentartrat aus.

In dem Salz nach einem der Aspekte 3 oder 4 der Erfindung ist die chirale Säure bevorzugt ausgewählt aus: (1S,4R)-Campher-10-sulfonsäure, (1R,4S)-Campher-10-sulfonsäure, (2R,3R)-Di-p-toluoylweinsäure, (2S,3S)-Di-p-toluoylweinsäure, (2R,3R)-Weinsäure, (2S,3S)-Weinsäure, (2R,3R)-Dibenzoylweinsäure und (2S,3S)-Dibenzoylweinsäure.

In dem Salz nach einem der Aspekte 3 oder 4 der Erfindung ist die chirale Säure bevorzugt ausgewählt aus: (1S,4R)-Campher-10-sulfonsäure, (1R,4S)-Campher-10-sulfonsäure, (2R,3R)-Di-p-toluoylweinsäure, (2S,3S)-Di-p-toluoylweinsäure, (2R,3R)-Weinsäure, (2S,3S)-Weinsäure, (2R,3R)-Dibenzoylweinsäure.

In dem Salz nach einem der Aspekte 3 oder 4 der Erfindung, einschließlich all den genannten Varianten bezüglich der chiralen Säure, ist die Verbindung der Formel **5** bevorzugt ausgewählt aus: (1R,5S)-Anhydroecgoninethylester, (1R,5S)-Anhydroecgoninmethylester, (1 R,5S)-Anhydroecgoninpropylester und (1R,5S)-Anhydroecgoninbutylester.

Das Salz nach einem der Aspekte 3 oder 4 der Erfindung ist bevorzugt ausgewählt aus der Gruppe:
(1R,5S)-Anhydroecgoninethylester-(1'S,4'R)-campher-10-sulfonat;
(1R,5S)-Anhydroecgoninethylester-(1'R,4'S)-campher-10-sulfonat;
(1R,5S)-Anhydroecgoninethylester-(2'S,3'S)-di-p-toluoylhydrogentartrat;
(1R,5S)-Anhydroecgoninethylester-(2'R,3'R)-di-p-toluoylhydrogentartrat;
(1R,5S)-Anhydroecgoninethylester-(2'R,3'R)-hydrogentartrat-monohydrat;
(1R,5S)-Anhydroecgoninethylester-(2'S,3'S)-hydrogentartrat-monohydrat;
(1R,5S)-Anhydroecgoninethylester-(2'R,3'R)-hydrogentartrat;
(1R,5S)-Anhydroecgoninethylester-(2'S,3'S)-hydrogentartrat;
(1R,5S)-Anhydroecgoninethylester-(2'R,3'R)-dibenzoylhydrogentartrat;
(1R,5S)-Anhydroecgoninethylester-(2'S,3'S)-dibenzoylhydrogentartrat.

Das Salz nach einem der Aspekte 3 oder 4 der Erfindung ist bevorzugt ausgewählt aus der Gruppe:
(1R,5S)-Anhydroecgoninethytester-(1'S,4'R)-campher-10-sulfonat;
(1R,5S)-Anhydroecgoninethylester-(1'R,4'S)-campher-10-sulfonat ;
(1R,5S)-Anhydroecgoninethylester-(2'S,3'S)-di-p-toluoylhydrogentartrat;
(1R,5S)-Anhydroecgoninethylester-(2'R,3'R)-di-p-toluoylhydrogentartrat;
(1R,5S)-Anhydroecgoninethylester-(2'R,3'R)-hydrogentartrat-monohydrat;
(1R,5S)-Anhydroecgoninethylester-(2'S,3'S)-hydrogentartrat-monohydrat;
(1R,5S)-Anhydroecgoninethylester-(2'R,3'R)-hydrogentartrat;
(1R,5S)-Anhydroecgoninethylester-(2'S,3'S)-hydrogentartrat;
(1R,5S)-Anhydroecgoninethylester-(2'R,3'R)-dibenzoylhydrogentartrat.

Das Salz nach einem der Aspekte 3 oder 4 der Erfindung, einschließlich aller oben genannten Varianten und Bevorzugungen, weist bevorzugt eine Enantiomerenreinheit von über etwa 95%, bevorzugt über etwa 96%, besonders bevorzugt über etwa 98%, insbesondere über etwa 99%, ganz besonders bevorzugt über etwa 99,9% auf.

Das Salz nach einem der Aspekte 3 oder 4 der Erfindung, einschließlich aller oben genannten Varianten und Bevorzugungen, kann ein Solvat sein, bevorzugt ein Hydrat, stärker bevorzugt ein Monohydrat.

Ein fünfter Aspekt der Erfindung betrifft eine Lösung eines (1R,5S)-Anhydroecgoninesters der allgemeinen chemischen Formel **5,** wie unter dem ersten Aspekt der Erfindung beschrieben, in Toluol, Xylol (alle Isomere), Halogenbenzolen, aliphatischen Kohlenwasserstoffen (C₅ bis C₈), halogenhaltigen, aliphatischen Kohlenwasserstoffen (C₁ bis C₆), aliphatischen Ethern (C₄ bis C₈), Estern der Ameisensäure (C₂ bis C₇), Estern der Essigsäure (C₃ bis C₇) oder Nitrilen (C₂ bis C₅).

Bevorzugtes Lösungsmittel ist Toluol.

Dabei beträgt der Gehalt an (1R,5S)-Anhydroecgoninesters wenigstens 20 Gew.%, bevorzugt wenigstens 40 Gew.% und stärker bevorzugt wenigstens 60 Gew.%.

Ein sechster Aspekt der Erfindung betrifft eine Suspension bestehend aus einem Suspensionsmittel ausgewählt aus der Gruppe Toluol, Xylol (alle Isomere), Halogenbenzole, aliphatische Kohlenwasserstoffe (C₅ bis C₈), halogenhaltige, aliphatische Kohlenwasserstoffe (C₁ bis C₆), aliphatische Ether (C₄ bis C₈), C₂ - C₇ -Alkyl-Ester der Ameisensäure, Ester der Essigsäure (C₃ bis C₇) oder C₂ - C₅ -Alkyl-Nitrile, bevorzugt Toluol und einem Salz nach einem der Aspekte 3 oder 4 der Erfindung, einschließlich aller Varianten und Bevorzugungen davon.

Die nachfolgenden Beispiele dienen der Illustration exemplarisch durchgeführter Syntheseverfahren. Sie sind lediglich als mögliche, beispielhaft dargestellte Vorgehensweisen zu verstehen, ohne die Erfindung auf deren Inhalt zu beschränken.

### BEISPIELE

### Beispiel 1

### Herstellung von Anhydroecgoninethylester

Die Herstellung des Anhydroecgoninethylester gelingt z.B. durch literaturbekannte Umesterung des nach den z.B. gemäß der eingangs beschriebenen Verfahren hergestellten Anhydroecgoninmethylesters gemäß der nachfolgenden Reaktionsgleichung: 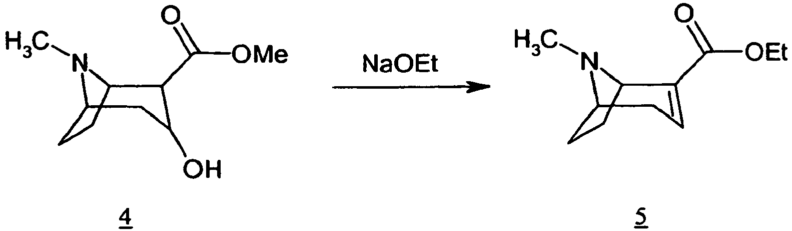 170 l 2-Carbomethoxytropinol-Konzentrat (Lösungsmittel: Essigsäureethylester; Gesamtalkaloidgehalt berechnet als 2-Carbomethoxytropinol: 34,4 kg, mittels HClO₄-Titration ermittelt) werden bei 52-58°C unter Vakuum auf ein Volumen von 60 l eingeengt und hierzu 350 l Ethanol gegeben. Die Lösung wird auf 18°C abgekühlt und bei dieser Temperatur mit 78,3 kg 21%ige Natriumethylatlösung versetzt. Das Reaktionsgemisch wird unter Rühren 1 h auf 52°C und dann 1 h auf 65°C erwärmt. Es wird auf 18°C abgekühlt und dann mit 34 l Eisessig versetzt. Das Reaktionsgemisch wird nun bei 51-58°C unter Vakuum auf 70 l eingeengt und 140 l Toluol hinzugegeben. Zur entstandenen Lösung fügt man 410 l Kondensat hinzu und stellt bei 21 °C mit 35 l 50%iger Schwefelsäure auf einen pH-Wert von 1,4 ein. Die beiden Phasen werden voneinander getrennt und die toluolische Phase verworfen. Die wässrige Phase wird mit 340 l Toluol versetzt und unter Rühren bei 24°C mit 54,5 l 50%iger Natronlauge auf einen pH-Wert von 8,5 eingestellt. Die Phasen werden getrennt und die wässrige Phase erneut mit 340 l Toluol extrahiert. Die vereinigten toluolischen Phasen werden mit 5,1 kg Natriumsulfat versetzt und nach 15 min 0,7 kg Aktivkohle und 0,34 kg Kieselgur zugegeben. Nach erfolgter Filtration wird das Lösungsmittel bei 50-58°C im Vakuum abdestilliert. Es verbleiben 30 l toluolisches Anhydroecgoninethylester-Konzentrat. Gehalt Stickstoff-Basen (HClO₄-Titration) 93,4%; Enantiomerenreinheit (chirale HPLC) 4,5% Fläche (1S,5R)-Anhydroecgoninethylester.

### Beispiele zur Herstellung von Salzen des (1R,5S)-Anhydroecgoninethylesters

Die Umsetzung mit chiralen Säuren wird anhand von mehreren Beispielen veranschaulicht.

### Beispiel 2:

### Herstellung von (1R,5S)-Anhydroecgoninethylester-(1'S,4'R)-campher-10-sulfonat

Die Umsetzung erfolgt anhand folgender Reaktionsgleichung: 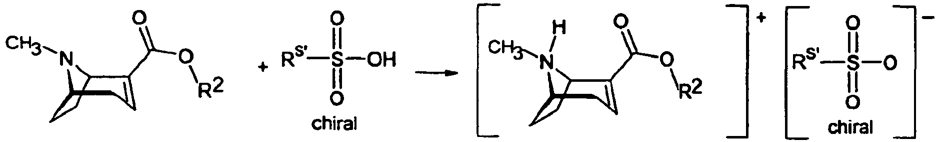 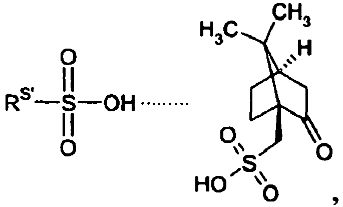 wobei R² = Ethyl ist.

23,8 g (1S,4R)-Campher-10-sulfonsäure werden in 120 ml Aceton bei 40°C gelöst und hierzu unter Rühren 25,3 g toluolisches Konzentrat von Anhydroecgoninethylester [Gehalt Stickstoff Basen (HClO₄-Titration) 79,1%; Chromatographische Reinheit (GC, ohne Toluol) 90,4% Fläche Anhydroecgoninethylester; Enantiomerenreinheit (chirale HPLC) 4,4% Fläche (1S,5R)-Anhydroecgoninethytester] zugegeben. Das Gemisch wird bei 40°C im Vakuum eingeengt und der verbliebene, ölige Rückstand bei Raumtemperatur in 30 ml Aceton aufgenommen. Anschließend wird mit einer kleinen Menge an Impfkristallen (ca. 0,1 g) versetzt, abgekühlt und die Temperatur 15 h bei 5°C gehalten. Die Endtemperatur beim Abscheiden wird vorzugsweise zwischen -15 und 10°C eingestellt.

Die entstandene Suspension wird über einen Büchnertrichter abgesaugt, der Niederschlag mit 20 ml kaltem Aceton gewaschen und anschließend 15 h bei 50°C im Vakuum (p = ca. 30 mbar) getrocknet. Das Abscheiden des Salzes erfolgt aus Aceton in Verdünnungen (Σ m_{Edukte} : Σ V_{Lösungsmittel}) von 2:1 bis 1:1. Als Waschflüssigkeit kommen neben Aceton auch lipophilere Lösungsmittel bzw. Lösungsmittelgemische in Frage.

Es werden 16,2 g des Campher-10-sulfonats in Form von weißen Kristallen erhalten. Smp. 136°C; Wassergehalt (Karl-Fischer-Titration) 0,1%; Gehalt Stickstoff-Basen (HClO₄-Titration, berechnet auf wasserfreie Substanz) 98,8%; Chromatographische Reinheit (GC, nach Basenfreisetzung) 98,8% Fl. Anhydroecgoninethylester; Enantiomerenreinheit (chirale HPLC) 99,9% Fl. (1R,5S)-Anhydroecgoninethylester / 0,1% Fl. (1S,5R)-Anhydroecgoninethylester.

### Beispiel 3:

### Herstellung von (1R,5S)-Anhydroecgoninethylester-(2'S,3'S)-di-p-toluoylhydrogen-tartrat

Die Umsetzung erfolgt anhand folgender Reaktionsgleichung: 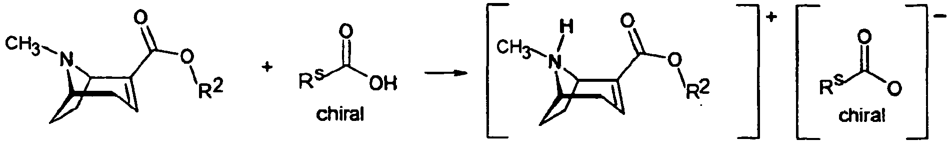 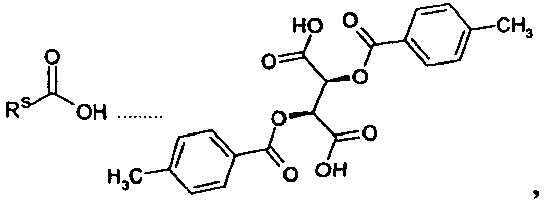 wobei R² = Ethyl ist.
36,1 g (2S,3S)-Di-p-toluoylweinsäure werden in 120 ml Acetonitril bei 50°C gelöst und hierzu rasch unter Rühren 22,8 g toluolisches Konzentrat von Anhydroecgoninethylester [Gehalt Stickstoff-Basen (HClO₄-Titration) 79,1%; Chromatographische Reinheit (GC, ohne Toluol) 90,4% Fl. Anhydroecgoninethylester; Enantiomerenreinheit (chirale HPLC) 4,4% Fl. (1S,SR)-Anhydroecgoninethylester] zugegeben. Die Lösung wird unter Rühren innerhalb von 2 h auf Raumtemperatur abgekühlt. Zu Beginn der Abkühlphase wird eine kleine Menge an Impfkristallen (ca. 0,1 g) zugesetzt. Die Endtemperatur beim Abscheiden liegt vorzugsweise zwischen 5 und 35°C. Das Abscheiden des Salzes erfolgt besonders bevorzugt aus Acetonitril in Verdünnungen (Σ m_{Edulkte} : Σ V_{Lösungsmittel}) von 1:1 bis 1:3. Man lässt 2 h nachrühren und trennt den ausgefallenen Niederschlag mittels Vakuumfiltration ab. Der Niederschlag wird mit 20 ml Acetonitril bei Raumtemperatur gewaschen und anschließend 15 h bei 50°C im Vakuum (p = ca. 30 mbar) getrocknet. Als Waschflüssigkeit kommen anstelle von Acetonitril auch lipophilere Lösungsmittel bzw. Lösungsmittelgemische in Frage. Zum Trennen von ausgefallenem Feststoff und Mutterlauge können übliche Vorrichtungen verwendet werden, beispielsweise Nutsche, Zentrifuge, Dekanter, Druckfilter etc. Es werden 41,3 g des Di-p-Toluoylhydrogentartrats in Form eines weißen Feststoffes erhalten. Smp. 142°C; Wassergehalt (Karl-Fischer-Titration) 1,0%; Gehalt Stickstoff-Basen (HClO₄-Titration, berechnet auf wasserfreie Substanz) 100,4%; Chromatographische Reinheit (GC, nach Basenfreisetzung) 98,1% Fl. Anhydroecgoninethylester; Enantiomerenreinheit (chirale HPLC) 99,4% Fl. (1R,5S)-Anhydroecgoninethylester / 0,6% Fl. (1S,5R)-Anhydroecgoninethylester.

### Beispiel 4:

### Herstellung von (1R,5S)-Anhydroecgoninethylester-(2'R,3'R)-hydrogentartrat-mono-hydrat

Die Umsetzung erfolgt anhand folgender Reaktionsgleichung: 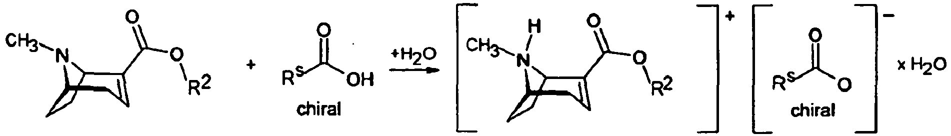 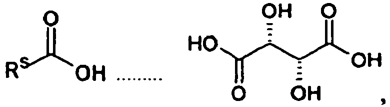 wobei R² = Ethyl ist.

Ansatz A: 43,9 g (2R,3R)-Weinsäure werden in einem Gemisch aus 15 ml H₂O und 300 ml Aceton aufgenommen, 5 min unter Rückfluss erhitzt und die entstandene Lösung heiß filtriert. 63,2 g toluolisches Konzentrat von Anhydroecgoninethylester [Gehalt Stickstoff-Basen (HClO₄-Titration) 79,1%; Chromatographische Reinheit (GC, ohne Toluol) 90,4% Fl. Anhydroecgoninethylester, Enantiomerenreinheit (chirale HPLC) 4,4% Fl. (1S,5R)-Anhydroecgoninethylester] werden bei Raumtemperatur in 200 ml Aceton aufgenommen, filtriert und anschließend zu der Weinsäure-Lösung gegeben. Die vereinigten Filtrate werden 10 min unter Rückfluss erhitzt und unter Rühren innerhalb von 3 h auf 20°C abgekühlt. Zu Beginn der Abkühlphase wird eine kleine Menge an Impfkristallen (ca. 0,1 g) zugesetzt. Man lässt bei 20°C 2 h nachrühren und trennt die ausgefallenen Kristalle mittels Vakuumfiltration ab. Die Endtemperatur beim Abscheiden liegt vorzugsweise zwischen 5 und 35°C. Das Abscheiden des Salzes wird bevorzugt aus Aceton/H₂O in Verdünnungen (Σ m_{Edukte} : Σ V_{Lösungsmittel}) von 1:2 bis 1:8, Lösungsmittelverhältnis Aceton/ H₂O 10 : 0.1 bis 10 : l durchgeführt. Das Trennen von ausgefallenem Feststoff und Mutterlauge erfolgt mit üblichen Vorrichtungen, wie Nutsche, Zentrifuge, Dekanter, Druckfilter etc. Falls für die Filtration der Edukte erforderlich können Filter- und Aufsaugmassen verwendet werden. Die Kristalle werden zweimal mit je 100 ml Aceton bei Raumtemperatur gewaschen und anschließend 15 h bei 50°C im Vakuum (p = ca. 30 mbar) getrocknet. Man erhält 73,9 g des Hydrogentartrat-Salzes in Form eines Monohydrates. Smp. 86°C; Wassergehalt (Karl-Fischer-Titration) 5,1%; Gehalt Stickstoff-Basen (HClO₄-Titration, berechnet auf wasserfreie Substanz) 99,9%; Chromatographische Reinheit (GC, nach Basenfreisetzung) 99,0% Fl. Anhydroecgoninethylester; Enantiomerenreinheit (chirale HPLC) 99,8% Fl. (1R,5S)-Anhydroecgoninethylester / 0,2% Fl. (1S,5R)-Anhydroecgoninethylester.

Ansatz B: 80,0 g (2R,3R)-Weinsäure werden in einem Gemisch aus 600 ml Aceton und 40 ml H₂O bei Raumtemperatur aufgenommen und die entstandene Lösung filtriert. Der Filter wird mit 10 ml Aceton nachgewaschen und das Filtrat auf 55°C erwärmt. 126,4 g toluolisches Konzentrat von Anhydroecgoninethylester [Gehalt Stickstoff-Basen (HClO₄-Titration) 79,1 %; Chromatographische Reinheit (GC, ohne Toluol) 90,4% Fl. Anhydroecgoninethylester; Enantiomerenreinheit (chirale HPLC) 4,4% Fl. (1S,5R)-Anhydroecgoninethylester] werden bei Raumtemperatur in 400 ml Aceton aufgenommen, filtriert und anschließend zu der erwärmten Weinsäure-Lösung gegeben. Die vereinigten Filtrate werden 10 min unter Rückfluss erhitzt und dann unter Rühren innerhalb von 3 h auf 20°C abgekühlt. Zu Beginn der Abkühlphase wird eine kleine Menge an Impfkristallen (ca. 0,1 g) zugesetzt. Man lässt bei 20°C 72 h nachrühren und trennt die ausgefallenen Kristalle mittels Vakuumfiltration ab. Die Endtemperatur beim Abscheiden liegt vorzugsweise zwischen 5 und 35°C. Das Abscheiden des Salzes wird bevorzugt aus Aceton/ H₂O in Verdünnungen (Σ m_{Edukte} : Σ V_{Lösungsfnittel}) von 1:2 bis 1:8, Lösungsmittelverhältnis Aceton/H₂O 10 : 0.1 bis 10 : 1 durchgeführt. Das Trennen von ausgefallenem Feststoff und Mutterlauge erfolgt mit üblichen Vorrichtungen, wie Nutsche, Zentrifuge, Dekanter, Druckfilter etc. Falls für die Filtration der Edukte erforderlich können Filter- und Aufsaugmassen verwendet werden. Die Kristalle werden zweimal mit je 200 ml Aceton bei Raumtemperatur gewaschen und anschließend 15 h bei 50°C im Vakuum (p = 30 mbar) getrocknet. Man erhält 150,7 g des Hydrogentartrat-Monohydrates in Form von weißen Kristallen. Smp. 85°C; Wassergehalt (Karl-Fischer-Titration) 5,3%; Gehalt Stickstoff-Basen (HClO₄-Titration, berechnet auf wasserfreie Substanz) 100,3%; Chromatographische Reinheit (GC, nach Basenfreisetzung) 99,0% Fl. Anhydroecgoninethylester; Enantiomerenreinheit (chirale HPLC) 99,8% Fl. (1R,5S)-Anhydroecgoninethylester / 0,2% Fl. (1S,5R)-Anhydroecgoninethylester.

### Beispiel 5:

### Herstellung von (1R,5S)-Anhydroecgoninethylester-(2'R,3'R)-dibenzoylhydrogen-tartrat

Die Umsetzung erfolgt anhand folgender Reaktionsgleichung: 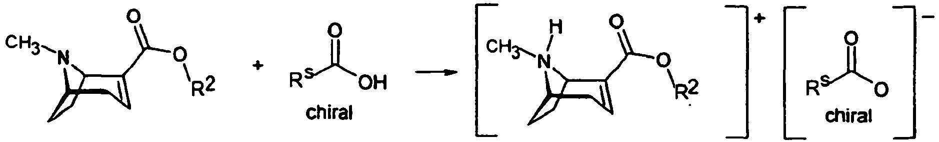 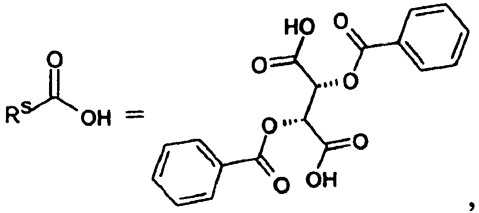 wobei R² = Ethyl ist.

83,5 g (2R,3R)-Dibenzoylweinsäure werden in 300 ml Ethanol 30 min unter Rückfluss erhitzt. Zu der entstandenen klaren Lösung gibt man unter Rühren 48,9 g toluolisches Konzentrat von Anhydroecgoninethylester [Gehalt Stickstoff-Basen (HClO₄-Titration) 93,1%; Chromatographische Reinheit (GC, ohne Toluol) 94,7% Fl. Anhydroecgoninethylester; Enantiomerenreinheit (chirale HPLC) 4.5% Fl. (1S,5R)-Anhydroecgoninethylester] und kocht die Lösung 5 min unter Rückfluss. Anschließend lässt man unter Rühren innerhalb von 2 h auf 20°C abkühlen, wobei ein Niederschlag ausfällt. Die Suspension wird weitere 2 h bei 20°C gerührt und dann über einen Büchnertrichter abgesaugt. Die Endtemperatur beim Abscheiden licgt vorzugsweise zwischen 5 und 35°C. Das Abscheiden des Salzes aus Ethanol wird vorzugsweise in Verdünnungen (Σ m_{Edukte} : Σ V_{Lösungsmittel}) von 1:1 bis 1:6 durchgeführt. Das Trennen von ausgefallenem Feststoff und Mutterlauge erfolgt mittels üblicher Vorrichtungen, wie beispielsweise einer Nutsche, Zentrifuge, einem Dekanter, einem Druckfilter etc. Der Niederschlag wird zweimal mit je 50 ml Ethanol bei Raumtemperatur gewaschen und anschließend 15 h bei 50 °C im Vakuum (p = ca. 30 mbar) getrocknet. Man erhält 120,1 g des Dibenzoylhydrogentartrats in Form eines weißen Feststoffes. Smp. 149°C, Wassergehalt (Karl-Fischer-Titration) 0,2%; Gehalt Stickstoff-Basen (HClO₄-Titration, berechnet auf wasserfreie Substanz) 99,8%; Chromatographische Reinheit (GC, nach Basenfreisetzung) 98,2% Fl. Anhydroecgoninethylester; Enantiomerenreinhcit (chirale HPLC) 97,3% Fl. (1R,5S)-Anhydroecgoninethylester / 2,7% Fl. (1S,5R)-Anhydroecgoninethylester.

## Patentansprüche

1. Verfahren zur Herstellung von chiralen Salzen von (1R,5S)-Anhydroecgoninestern der allgemeinen chemischen Formel **5** in bevorzugt enantiomeren-angereicherter Form: 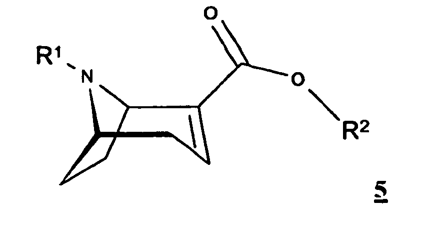 worin
R¹ Wasserstoff, einen Alkylrest, bevorzugt Methyl, Ethyl, Propyl oder Butyl, oder eine beliebige Schutzgruppe, bevorzugt Allyl, Benzyl, Methoxybenzyl, Allyloxycarbonyl, Benzyloxycarbonyl, tert.-Butyloxycarbonyl, 9-Fluorenylmethyloxycarbonyl, Acetyl, Benzoyl oder Formyl, darstellt;
R² Alkyl, Aryl, bevorzugt Phenyl oder Naphthyl, gegebenenfalls substituiert mit ein oder mehreren Substituenten ausgewählt aus Halogen, Hydroxy, Amino, Cyano, Nitro, Trifluormethyl, Trifluormethoxy, Alkoxy, Cycloalkoxy, Alkyl, Cycloalkyl, Cycloalkylalkyl, Alkenyl und Alkinyl, darstellt; bevorzugt ist R² Methyl, Ethyl, Propyl oder Butyl
mit den Schritten
(1a) Umsetzen einer Verbindung der Formel **1** mit Methanol und ggf. in Methanol als Lösungsmittel zu einer Verbindung der Formel **1'**: 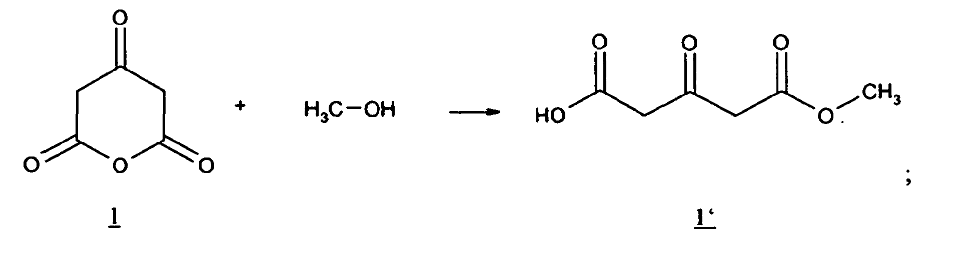
(1b) Umsetzen einer Verbindung der Formel **2** mit Wasser und Katalysator zu einer Verbindung der Formel **2'** 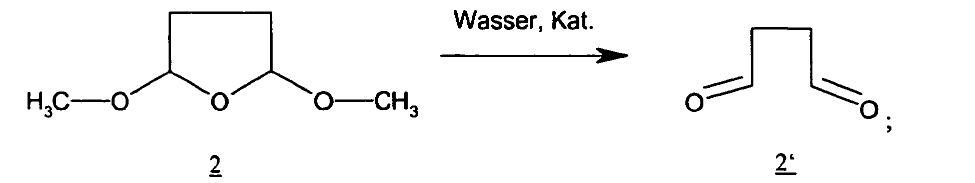
(2) Umsetzen einer Verbindung der Formel **1'** und einer Verbindung der Formel **2'** mit einer R¹-Amin-Lösung zu einer Verbindung der Formel **3**: 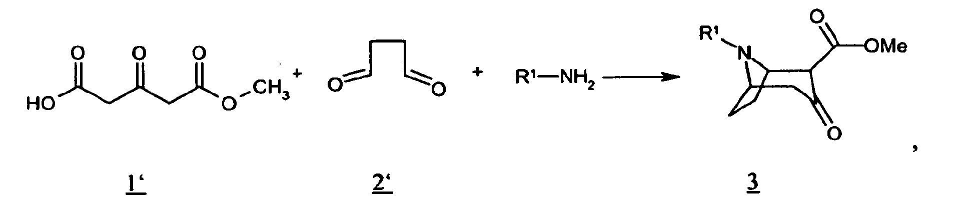 worin R¹ Wasserstoff, einen Alkylrest, bevorzugt Methyl, Ethyl, Propyl oder Butyl, oder eine beliebige Schutzgruppe, bevorzugt Allyl, Benzyl, Methoxybenzyl, Allyloxycarbonyl, Benzyloxycarbonyl, tert.-Butyloxycarbonyl, 9-Fluorenylmethyloxycarbonyl, Acetyl, Benzoyl oder Formyl, darstellt;
(3) optional Umsetzen der Verbindung der Formel **3** mit (2R,3R)-Weinsäure [L(+)-Weinsäure] unter Anreicherung des (R)-Enantiomer-Hydrogentartratsalzes der Verbindung der Formel **3'**: 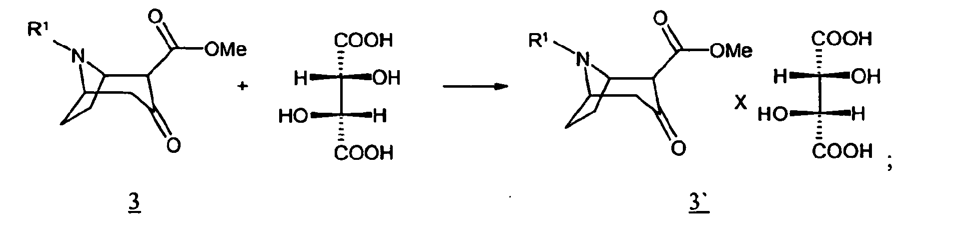
(4) optional Freisetzung der Verbindung **4** im Basischen 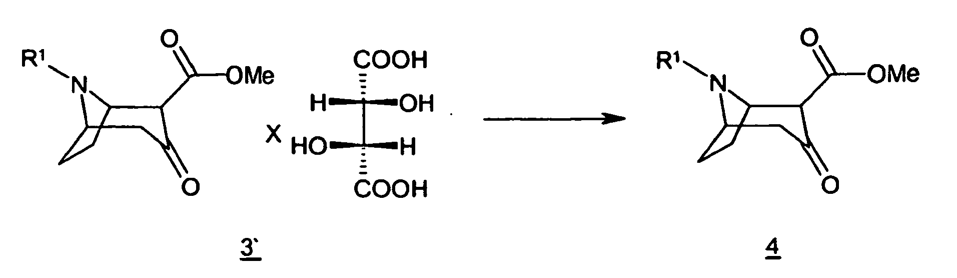
(5) Reduktion des erhaltenen Produkts und ggf. weitere Umesterung mit einem Alkoholat der Formel MOR² zu einer Verbindung der Formel **5**: 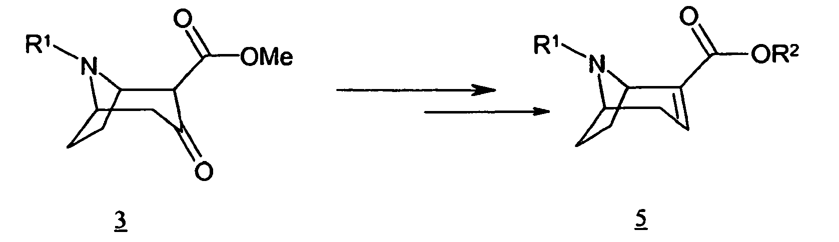 worin R² Alkyl, Aryl, bevorzugt Phenyl oder Naphthyl, gegebenenfalls substituiert mit ein oder mehreren Substituenten ausgewählt aus Halogen, Hydroxy, Amino, Cyano, Nitro, Trifluormethyl, Trifluormethoxy, Alkoxy, Cycloalkoxy, Alkyl, Gycloalkyl, Cycloalkylalkyl, Alkenyl und Alkinyl, darstellt, R² bevorzugt Methyl, Ethyl, Propyl oder Butyl
darstellt,
M ein Alkali- oder Erdalkalimetall, bevorzugt Kalium oder Natrium, darstellt; und
(6) Umsetzen der Verbindung der Formel **5** mit einer chiralen Säure zu einem Salz der Verbindung der Formel **5**': 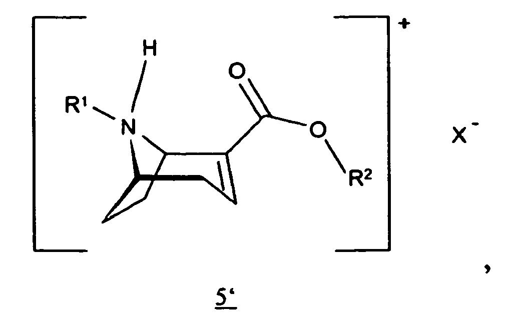 wobei X⁻ das Anion einer chiralen Säure darstellt, welche bevorzugt ausgewählt ist aus der Gruppe (1S,4R)-Campher-10-sulfonsäure, (1R,4S)-Campher-10-sulfonsäure, (2R,3R)-Di-p-toluoylweinsäure, (2S,3S)-Di-p-toluoylweinsäure, (2R,3R)-Weinsäure, (2S,3S)-Weinsäure, (2R,3R)-Dibenzoylweinsäure und (2S,3S)-Dibenzoylweinsäure;
(7) ggf. Aufreinigung der chiralen Verbindung der Formel **5**' und
(8) ggf. Kristallisation.

2. Verfahren nach Anspruch 1, **dadurch gekennzeichnet, dass** am Ende der Aufarbeitung in Schritt (5) durch Zugabe von Toluol eine toluolische konzentrierte Lösung der Verbindung der Formel **5** hergestellt wird

3. Verfahren nach Anspruch 1, **dadurch gekennzeichnet, dass** im Schritt (6) die Verbindung der Formel **5** in Toluol als Konzentrat gelöst zu wenigstens 20 Gew.% vorliegt, bevorzugt zu wenigstens 40 Gew.%, besonders bevorzugt zu wenigstens 60 Gew.%.

4. Verfahren nach Anspruch 1, **dadurch gekennzeichnet, dass** in Schritt (6) die chirale Säure in einem Lösungsmittel vorgelegt und hierzu die toluolische konzentrierte Lösung der Verbindung der Formel **5** zugegeben wird.

5. Verfahren nach Anspruch 1, **dadurch gekennzeichnet, dass** das Lösungsmittel in Schritt (6) ausgewählt wird aus polaren bzw. mittelpolaren protischen oder aprotischen Lösungsmitteln, bevorzugt Aceton, C₁- bis C₅-Alkoholen, C₂- bis C₃-Nitrilen, C₃- bis C₆-Ketonen, mit oder ohne Zusatz von Wasser.

6. Verfahren nach Anspruch 1, **dadurch gekennzeichnet, dass** die chirale Säure im Lösungsmittel in Schritt (6) unter Erhitzen auf eine Temperatur im Bereich von etwa 35°C bis etwa zur Rückflusstemperatur des verwendeten Lösungsmittels gelöst wird, die Verbindung der Formel **5** in Form einer toluolischen konzentrierten Lösung oder Suspension zu der Lösung der chiralen Säure bei oder in der Nähe der Lösungstemperatur der chiralen Säure zugesetzt wird, nach Zugabe der toluolischen konzentrierten Lösung oder Suspension und gegebenenfalls Erhitzen bis auf Rückflusstemperatur des Lösungsmittels, zum Ausfällen der Verbindung der Formel **5**' das Gemisch abgekühlt wird, bevorzugt bis zu einer Endtemperatur zwischen -15 und 35°C, bevorzugt, 5 bis 35°C und der Ausfällungsvorgang, ggf. durch Animpfen mit einer kleinen Menge an Impfkristallen und/oder Anreiben unterstützt wird.

7. Verfahren zur Herstellung von Salzen der Verbindung der Formel **5**: 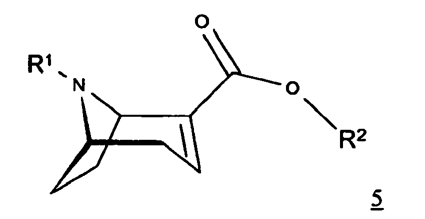 wobei die Verbindung der Formel **5** mit einer chiralen Säure zu einem Salz der Verbindung der Formel **5**' umgesetzt wird: 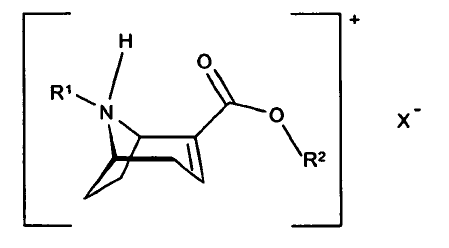 wobei R¹ und R² wie in Anspruch 1 definiert sind, und
X⁻ das Anion einer chiralen Säure darstellt, bevorzugt ausgewählt aus der Gruppe (1S,4R)-Campher- 10-sulfonsäure, (1R,4S)-Campher- 10-sulfonsäure, (2R,3R)-Di-p-toluoylwclnsäure, (2S,3S)-Di-p-totuoylweinsäure, (2R,3R)-Weinsäure, (2S,3S)-Weinsäure, (2R,3R)-Dibenzoylweinsäure und (2S,3S)-Dibenzoylweinsäure.

8. Verfahren nach Anspruch 7, **dadurch gekennzeichnet, dass** Bedingungen denen nach einem der Ansprüche 2 bis 6 entsprechen.

9. Verfahren nach mindestens einem der vorangehenden Ansprüche 7 oder 8, **dadurch gekennzeichnet, dass** die Verbindung der Formel **5**' in Form eines Enantiomeren in einer Enantiomerenreinheit von über etwa 95%, bevorzugt über etwa 96%, besonders bevorzugt über etwa 98%, insbesondere über etwa 99%, ganz besonders bevorzugt über etwa 99,9% ausgefällt wird.

10. Enantiomeremeines Salz der Verbindung der Formel **5** 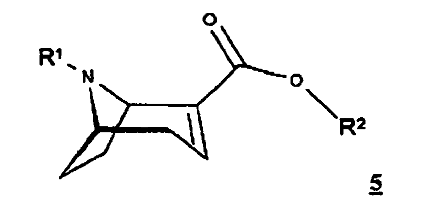 worin
R¹ einen Alkylrest, bevorzugt methyl, Ethyl, Propyl oder Butyl, oder eine beliebige Schutzgruppe, bevorzugt Allyl, Benzyl, Methoxybenzyl, Allyloxycarbonyl, Benzyloxycarbonyl, tert.-Butyloxycarbonyl, 9-Fluorenylmethyloxycarbonyl, Acetyl, Benzoyl oder Formyl, darstellt;
R² Alkyl, Aryl, bevorzugt Phenyl oder Naphthyl, gegebenenfalls substituiert mit ein oder mehreren Substituenten ausgewählt aus Halogen, Hydroxy, Amino, Cyano, Nitro, Trifluormethyl, Trifluormethoxy, Alkoxy, Cycloalkoxy, Alkyl, Cycloalkyl, Cycloalkylalkyl, Alkenyl und Alkinyl, darstellt; bevorzugt ist R² Methyl, Ethyl, Propyl oder Butyl
mit einer chiralen Säure, welches nicht (1R,5S)-Anhydroecgoninethylester-(2`S,3'S)-dibenzoylhydrogentartrat ist.

11. Chirales, bevorzugt enantiomerenreines Salz der Verbindung der Formel **5** 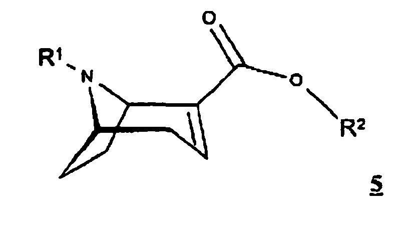 worin
R¹ einen Alkylrest, bevorzugt Methyl, Ethyl, Propyl oder Butyl, oder eine beliebige Schutzgruppe, bevorzugt Allyl, Benzyl, Methoxybenzyl, Allyloxycarbonyl, Benzyloxycarbonyl, tert.-Butyloxycarbonyl, 9-Fluorenylmethyloxycarbonyl, Acetyl, Benzoyl oder Formyl, darstellt;
R² Alkyl, Aryl, bevorzugt Phenyl oder Naphthyl, gegebenenfalls substituiert mit ein oder mehreren Substituenten ausgewählt aus Halogen, Hydroxy, Amino, Cyano, Nitro, Trifluormethyl, Trifluormethoxy, Alkoxy, Cycloalkoxy, Alkyl, Cycloalkyl, Cycloalkylalkyl, Alkenyl und Alkinyl, darstellt; bevorzugt ist R² Methyl, Ethyl, Propyl oder Butyl
mit einer chiralen Säure, welches kristallin und nicht (1R,5S)-Anhydroecgoninethylester-(2'S,3'S)-dibenzoylhydrogentartrat ist.

12. Salz nach Anspruch 10 oder 11, **dadurch gekennzeichnet, dass** die chirale Säure ausgewählt ist aus: (1S,4R)-Campher-10-sulfonsäure, (1R,4S)-Campher-10-sulfonsäure, (2R,3R)-Di-p-toluoylweinsäure, (2S,3S)-Di-p-toluoylweinsäure, (2R,3R)-Weinsäure, (2S,3S)-Weinsäure, (2R,3R)-Dibenzoylweinsäure und (2S,3S)-Dibenzoylweinsäure und die Verbindung der Formel **5** ausgewählt ist aus (1R,5S)-Anhydroecgoninethylester, (1R,5S)-Anhydroecgoninmethylester, (1R,5S)-Anhydroecgoninpropylester und (1R,5S)-Anhydroecgoninbutylester.

13. Salz nach einem der Ansprüche 10 bis 12, **dadurch gekennzeichnet, dass** das Salz eine der nachfolgenden Verbindungen darstellt:
(1R,5S)-Anhydroecgoninethylester-(1'S,4'R)-campher-10-sulfonat;
(1R,5S)-Anhydroecgoninethylester-(1'R,4'S)-campher-10-sulfonat;
(1R,5S)-Anhydroecgoninethylester-(2'S,3'S)-di-p-toluoylhydrogentartrat;
(1R,5S)-Anhydroecgoninethylester-(2'R,3'R)-di-p-toluoylhydrogentartrat;
(1R, 5S)-Anhydroecgoninethylester-(2'R,3'R)-hydrogentartrat-monohydrat;
(1R,5S)-Anhydroecgoninethylester-(2'S,3'S)-hydrogentartrat-monohydrat;
(1R,5S)-Anhydroecgoninethylester-(2'R,3'R)-hydrogentartrat;
(1R,5S)-Anhydroecgoninethylester-(2'S,3'S)-hydrogentartrat;
(1R,5S)-Anhydroecgoninethylester-(2'R,3 R)-dibenzoylhydrogentartrat,
(1R,5S)-Anhydroecgoninethylester-(2'S,3''S)-dibenzoylhydrogentartrat, bevorzugt
(1R, 5S)-Anhydroecgoninethylester-(1'S, 4'R)-campher-10-sulfonat;
(1R,5S)-Anhydroecgoninethylester-(1'R,4'S)-campher-10-sulfonat;
(1R,5S)-Anhydroecgoninethylester-(2'S,3'S)-di-p-toluoylhydrogentartrat;
(1R,5S)-Anhydroecgoninethylester-(2'R,3'R)-di-p-toluoylhydrogentartrat;
(1R,5S)-Anhydroecgoninethylester-(2'R,3'R)-hydrogentartrat-monohydrat;
(1R,5S)-Anhydroecgoninethylester-(2'S,3'S)-hydrogentartrat-monohydrat;
(1R,5S)-Anhydroecgoninethylester-(2'R,3'R)-hydrogentartrat;
(1R,5S)-Anhydroecgoninethylester-(2'S,3'S)-hydrogentartrat;
(1R,5S)-Anhydroecgoninethylester-(2'R,3'R)-dibenzoylhydrogentartrat.

14. Lösung eines (1R,5S)-Anhydroecgoninesters der allgemeinen chemischen Formel **5** nach Anspruch 1 mit einem Gehalt von wenigstens 20 Gew.%, bevorzugt wenigstens 40 Gew.% und stärker bevorzugt wenigstens 60 Gew. % in Toluol, Xylol (alle Isomere), Halogenbenzolen, aliphatischen Kohlenwasserstoffen (C₅ bis C₈), halogenhaltigen, aliphatischen Kohlenwasserstoffen (C₁ bis C₆), aliphatischen Ethern (C₄ bis C₈), Estern der Ameisensäure (C₂ bis C₇), Estern der Essigsäure (C₃ bis C₇) oder Nitrilen (C₂ bis C₅), bevorzugt Toluol.

15. Suspension bestehend aus einem Suspensionssmittel ausgewählt aus der Gruppe Toluol, Xylol (alle Isomere), Halogenbenzole, aliphatische Kohlenwasserstoffe (C₅ bis C₈), halogenhaltige, aliphatische Kohlenwasserstoffe (C₁ bis C₆), aliphatische Ether (C₄ bis C₈), C₂ - C₇ -Alkyl-Ester der Ameisensäure, Ester der Essigsäure (C₃ bis C₇) oder C₂ - C₅ Alkyl-Nitrile, bevorzugt Toluol und einem Salz nach einem der Ansprüche 10 bis 13.

## Claims

1. Process for preparing chiral salts of (1R,5S)-anhydroecgonine esters of the general chemical formula **5** in preferably enantiomer-enriched form: 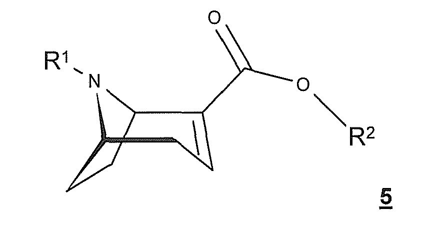 wherein
R¹ denotes hydrogen, an alkyl group, preferably methyl, ethyl, propyl or butyl, or any desired protective group, preferably allyl, benzyl, methoxybenzyl, allyloxycarbonyl, benzyloxycarbonyl, tert.-butyloxycarbonyl, 9-fluorenylmethyloxycarbonyl, acetyl, benzoyl or formyl;
R² denotes alkyl, aryl, preferably phenyl or naphthyl, optionally substituted by one or more substituents selected from halogen, hydroxy, amino, cyano, nitro, trifluoromethyl, trifluoromethoxy, alkoxy, cycloalkoxy, alkyl, cycloalkyl, cycloalkylalkyl, alkenyl and alkynyl; preferably R² is methyl, ethyl, propyl or butyl
comprising the steps of
(1 a) reacting a compound of formula **1** with methanol and optionally in methanol as solvent to form a compound of formula **1**': 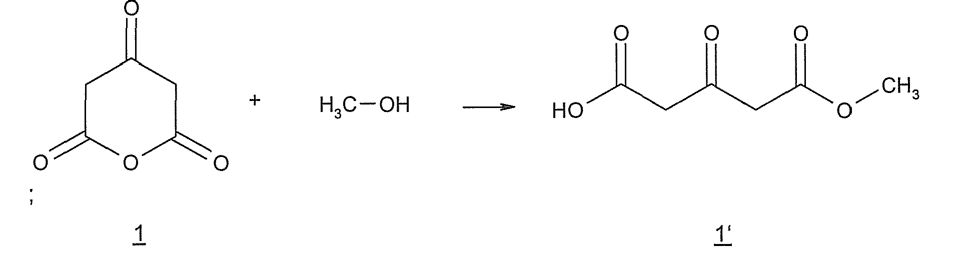
(1 b) reacting a compound of formula **2** with water and catalyst to form a compound of formula **2**': 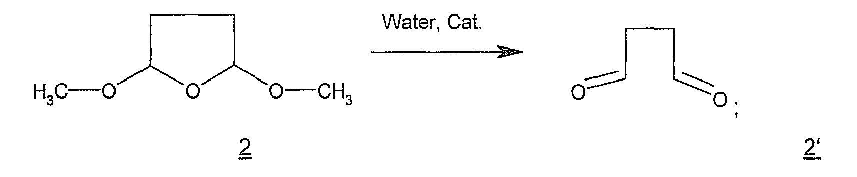
(2) reacting a compound of formula **1**' and a compound of formula **2**' with an R¹-amine solution to form a compound of formula **3**: 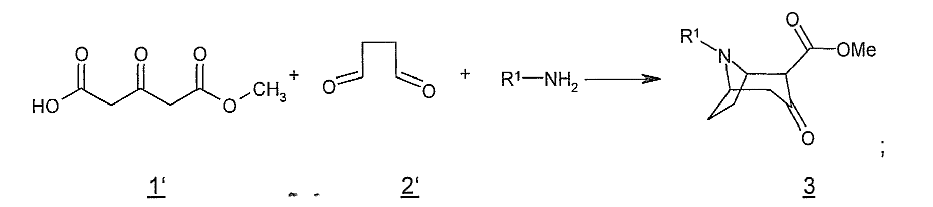 wherein R¹ denotes hydrogen, an alkyl group, preferably methyl, ethyl, propyl or butyl, or any desired protective group, preferably allyl, benzyl, methoxybenzyl, allyloxycarbonyl, benzyloxycarbonyl, tert.-butyloxycarbonyl, 9-fluorenylmethyloxycarbonyl, acetyl, benzoyl or formyl;
(3) optionally reacting the compound of formula **3** with (2R,3R)-tartaric acid [L(+)-tartaric acid] with enrichment of the (R)-enantiomer-hydrogen tartrate salt of the compound of formula **3**': 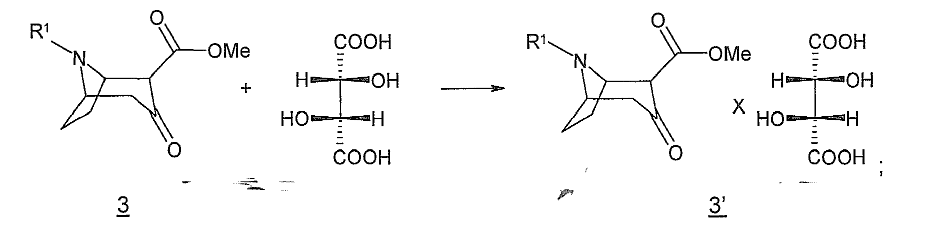
(4) optionally releasing compound **4** in the basic 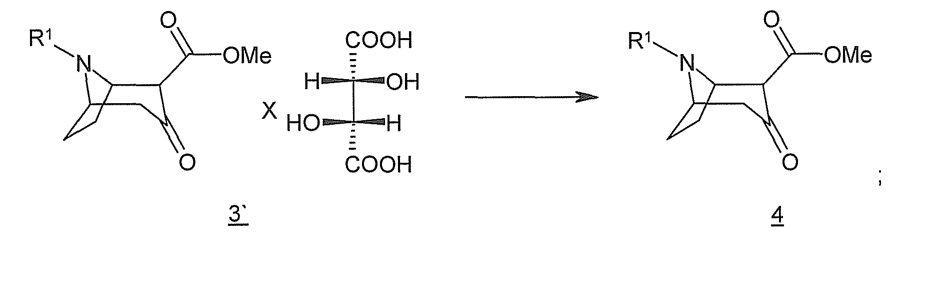
(5) reducing the product obtained and optionally carrying out further transesterification with an alkoxide of formula MOR² to obtain a compound of formula **5**: 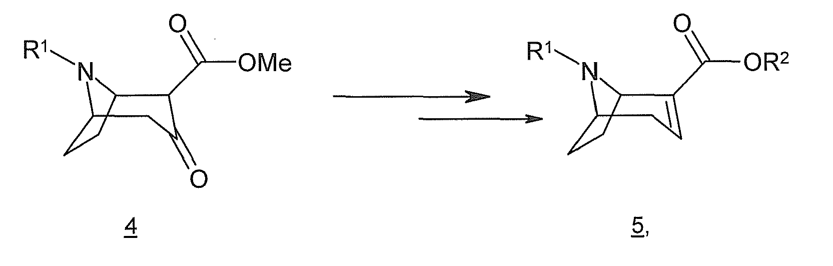 wherein R² denotes alkyl, aryl, preferably phenyl or naphthyl, optionally substituted by one or more substituents selected from halogen, hydroxy, amino, cyano, nitro, trifluoromethyl, trifluoromethoxy, alkoxy, cycloalkoxy, alkyl, cycloalkyl, cycloalkylalkyl, alkenyl and alkynyl, R² preferably denotes methyl, ethyl, propyl or butyl,
M denotes an alkali or alkaline earth metal, preferably potassium or sodium; and
(6) reacting the compound of formula **5** with a chiral acid to form a salt of the compound of formula **5**'; 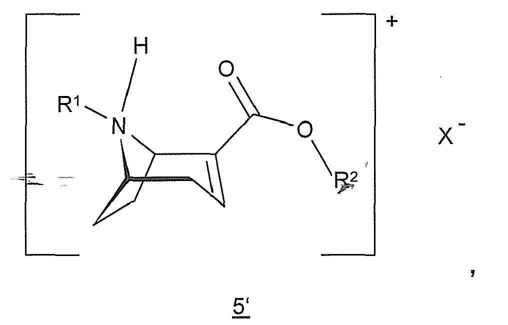 wherein X⁻ denotes the anion of a chiral acid which is preferably selected from among (1S,4R)-camphor-10-sulphonic acid, (1R,4S)-camphor-10-sulphonic acid, (2R,3R)-di-p-toluoyltartaric acid, (2S,3S)-di-p-toluoyltartaric acid, (2R,3R)-tartaric acid, (2S,3S)-tartaric acid, (2R,3R)-dibenzoyltartaric acid and (2S,3S)-dibenzoyltartaric acid;
(7) optionally purifying the chiral compound of formula **5'** and
(8) optionally carrying out crystallisation.

2. Process according to claim 1, **characterised in that** at the end of the working up in step (5) a concentrated toluene solution of the compound of formula **5** is prepared by the addition of toluene.

3. Process according to claim 1, **characterised in that** in step (6) the compound of formula **5** is present as a concentrate dissolved in toluene in an amount of at least 20 wt.%, preferably at least 40 wt.%, particularly preferably at least 60 wt.%.

4. Process according to claim 1, **characterised in that** in step (6) the chiral acid is placed in a solvent and to this is added the concentrated toluene solution of the compound of formula **5**.

5. Process according to claim 1, **characterised in that** the solvent in step (6) is selected from among polar or medium-polar protic or aprotic solvents, preferably acetone, C₁- to C₅-alcohols, C₂- to C₃-nitriles, C₃- to C₆-ketones, with or without the addition of water.

6. Process according to claim 1, **characterised in that** the chiral acid is dissolved in the solvent in step (6) with heating to a temperature in the range from about 35°C to approximately the reflux temperature of the solvent used, the compound of formula **5** is added in the form of a concentrated toluene solution or suspension to the solution of the chiral acid at or around the temperature of dissolution of the chiral acid, after the addition of the concentrated toluene solution or suspension and optionally heating to the reflux temperature of the solvent, in order to precipitate the compound of formula **5'** the mixture is cooled, preferably to a final temperature of between -15 and 35°C, preferably 5 to 35°C, and the precipitation process is optionally assisted by inoculation with a small amount of seed crystals and/or trituration.

7. Process for preparing salts of the compound of formula **5**: 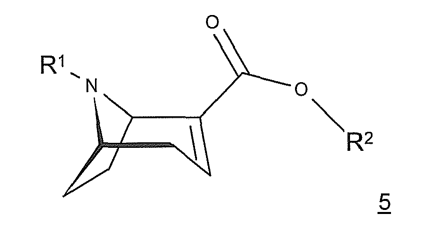 wherein the compound of formula **5** is reacted with a chiral acid to form a salt of the compound of formula **5**': 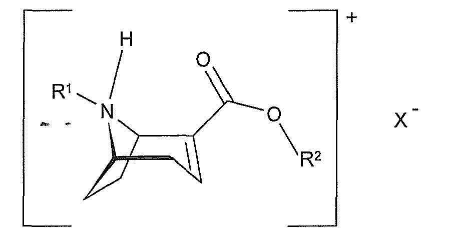 wherein R¹ and R² are defined as in claim 1, and
X- denotes the anion of a chiral acid, preferably selected from among (1 S,4R)-camphor-10-sulphonic acid, (1 R,4S)-camphor-10-sulphonic acid, (2R,3R)-di-p-toluoyltartaric acid, (2S,3S)-di-p-toluoyltartaric acid, (2R,3R)-tartaric acid, (2S,3S)-tartaric acid, (2R,3R)-dibenzoyltartaric acid and (2S,3S)-dibenzoyltartaric acid.

8. Process according to claim 7, **characterised in that** conditions correspond to those according to one of claims 2 to 6.

9. Process accordling to at least one of the preceding claims 7 or 8, **characterised in that** the compound of formula **5'** is precipitated in the form of an enantiomer in an enantiomeric purity of more than about 95%, preferably more than about 96%, particularly preferably more than about 98%, particularly more than about 99%, most particularly preferably more than about 99.9%.

10. Enantiomerically pure salt of the compound of formula **5** 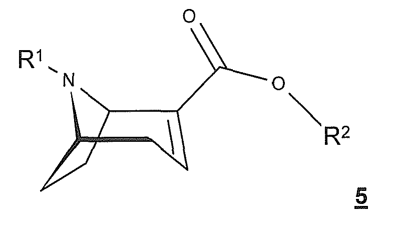 wherein
R¹ denotes an alkyl group, preferably methyl, ethyl, propyl or butyl, or any desired protective group, preferably allyl, benzyl, methoxybenzyl, allyloxycarbonyl, benzyloxycarbonyl, tert.-butyloxycarbonyl, 9-fluorenylmethyloxycarbonyl, acetyl, benzoyl or formyl;
R² denotes alkyl, aryl, preferably phenyl or naphthyl, optionally substituted by one or more substituents selected from halogen, hydroxy, amino, cyano, nitro, trifluoromethyl, trifluoromethoxy, alkoxy, cycloalkoxy, alkyl, cycloalkyl, cycloalkylalkyl, alkenyl and alkynyl; preferably R² is methyl, ethyl, propyl or butyl
with a chiral acid, which is not (1R,5S)-anhydroecgonine ethyl ester-(2'S,3'S)-dibenzoylhydrogen tartrate.

11. Chiral, preferably enantiomerically pure salt of the compound of formula **5** 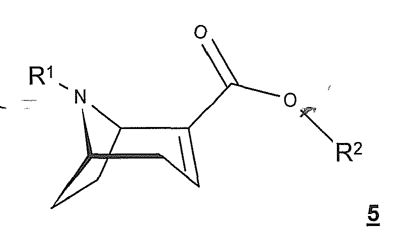 wherein
R¹ denotes an alkyl group, preferably methyl, ethyl, propyl or butyl, or any desired protective group, preferably allyl, benzyl, methoxybenzyl, allyloxycarbonyl, benzyloxycarbonyl, tert.-butyloxycarbonyl, 9-fluorenylmethyloxycarbonyl, acetyl, benzoyl or formyl;
R² denotes alkyl, aryl, preferably phenyl or naphthyl, optionally substituted by one or more substituents selected from halogen, hydroxy, amino, cyano, nitro, trifluoromethyl, trifluoromethoxy, alkoxy, cycloalkoxy, alkyl, cycloalkyl, cycloalkylalkyl, alkenyl and alkynyl; preferably R² is methyl, ethyl, propyl or butyl
with a chiral acid which is crystalline and is not (1R,5S)-anhydroecgonine ethyl ester-(2'S,3'S)-dibenzoylhydrogen tartrate.

12. Salt according to claim 10 or 11, **characterised in that** the chiral acid is selected from among: (1 S,4R)-camphor-10-sulphonic acid, (1R,4S)-camphor-10-sulphonic acid, (2R,3R)-di-p-toluoyltartaric acid, (2S,3S)-di-p-toluoyltartaric acid, (2R,3R)-tartaric acid, (2S,3S)-tartaric acid, (2R,3R)-dibenzoyltartaric acid and (2S,3S)-dibenzoyltartaric acid and the compound of formula **5** is selected from among (1R,5S)-anhydroecgonine ethyl ester, (1R,5S)-anhydroecgonine methyl ester, (1R,5S)-anhydroecgonine propyl ester and (1R,5S)-anhydroecgonine butyl ester.

13. Salt according to one of claims 10 to 12, **characterised in that** the salt is one of the following compounds:
(1R,5S)-anhydroecgonine ethyl ester-(1'S,4'R)-camphor-10-sulphonate;
(1R,5S)-anhydroecgonine ethyl ester-(1'R,4'S)-camphor-1 0-sulphonate;
(1R,5S)-anhydroecgonine ethyl ester-(2'S,3'S)-di-p-toluoylhydrogen tartrate;
(1R,5S)-anhydroecgonine ethyl ester-(2'R,3'R)-di-p-toluoylhydrogen tartrate;
(1R,5S)-anhydroecgonine ethyl ester-(2'R,3'R)-hydrogen tartrate-monohydrate;
(1R,5S)-anhydroecgonine ethyl ester-(2'S,3'S)-hydrogen tartrate-monohydrate;
(1R,5S)-anhydroecgonine ethyl ester-(2'R,3'R)-hydrogen tartrate;
(1R, 5S)-anhydroecgonine ethyl ester-(2'S,3'S)-hydrogen tartrate;
(1R,5S)-anhydroecgonine ethyl ester-(2'R,3'R)-dibenzoylhydrogen tartrate,
(1R,5S)-anhydroecgonine ethyl ester-(2'S, 3'S)-dibenzoylhydrogen tartrate, preferably
(1R,5S)-anhydroecgonine ethyl ester-(1'S,4'R)-camphor-10-sulphonate;
(1R,5S)-anhydroecgonine ethyl ester-(1'R,4'S)-camphor-10-sulphonate;
(1R,5S)-anhydroecgonine ethyl ester-(2'S,3'S)-di-p-toluoylhydrogen tartrate;
(1R,5S)-anhydroecgonine ethyl ester-(2'R,3'R)-di-p-toluoylhydrogen tartrate;
(1R,5S)-anhydroecgonine ethyl ester-(2'R,3'R)-hydrogen tartrate-monohydrate;
(1R,5S)-anhydroecgonine ethyl ester-(2'S,3'S)-hydrogen tartrate-monohydrate;
(1R,5S)-anhydroecgonine ethyl ester-(2'R,3'R)-hydrogen tartrate;
(1R,5S)-anhydroecgonine ethyl ester-(2'S,3'S)-hydrogen tartrate;
(1R,5S)-anhydroecgonine ethyl ester-(2'R,3'R)-dibenzoylhydrogen tartrate.

14. Solution of a (1R,5S)-anhydroecgonine ester of general chemical formula **5** according to claim 1 having a content of at least 20 wt.%, preferably at least 40 wt.% and more preferably at least 60 wt.% of toluene, xylene (all the isomers), halobenzenes, aliphatic hydrocarbons (C₅ to C₈), halogen-containing, aliphatic hydrocarbons (C₁ to C₆), aliphatic ethers (C₄ to C₈), esters of formic acid (C₂ to C₇), esters of acetic acid (C₃ to C₇) or nitriles (C₂ to C₅), preferably toluene.

15. Suspension consisting of a suspension agent selected from among toluene, xylene (all the isomers), halobenzenes, aliphatic hydrocarbons (C₅ to C₈), halogen-containing aliphatic hydrocarbons (C₁ to C₆), aliphatic ethers (C₄ to C₈), C₂ - C₇ - alkyl-esters of formic acid, esters of acetic acid (C₃ to C₇) or C₂ - C₅-alkyl-nitriles, preferably toluene and a salt according to one of claims 10 to 13.

## Revendications

1. Procédé de production de sels chiraux de (1R, 5S)-anhydroecgonine-esters de formule chimique générale 5 de préférence sous forme enrichie en énantiomères : 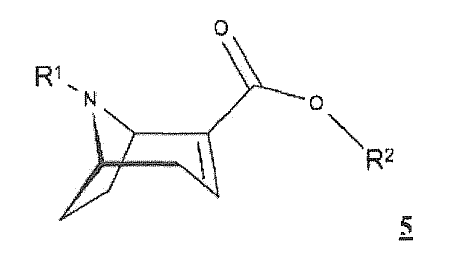 dans laquelle
R¹ est un atome d'hydrogène, un radical alkyle, de préférence, méthyle, éthyle, propyle ou butyle ou un groupe protecteur quelconque, de préférence allyle, benzyle, méthoxybenzyle, allyloxycarbonyle, benzyloxycarbonyle, tert-butyloxycarbonyle, 9-fluorénylméthyloxycarbonyle, acétyle, benzoyle ou formyle ;
R² est un groupe alkyle, aryle, de préférence phényle ou naphtyle, éventuellement substitué par un ou plusieurs substituants choisis parmi un atome d'halogène, un groupe hydroxy, amino, cyano, nitro, trifluorométhyle, trifluorométhoxy, alcoxy, cycloalcoxy, alkyle, cycloalkyle, cycloalkylalkyle, alcényle et alcinyle ; de préférence R² est un groupe méthyle, éthyle, propyle ou butyle
comprenant les étapes de
(1a) conversion d'un composé de formule 1 avec du méthanol et éventuellement dans du méthanol comme solvant, en un composé de formule 1' 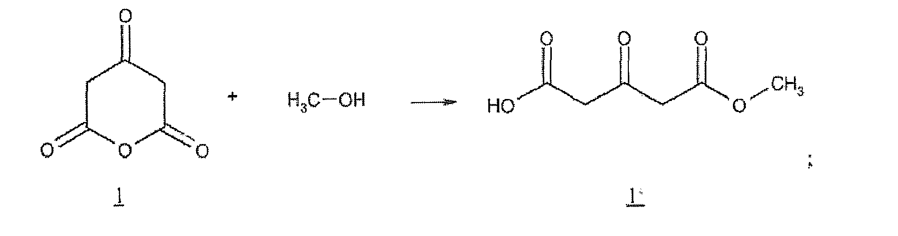
(1b) conversion d'un composé de formule 2 avec de l'eau et un catalyseur en un composé de formule 2' 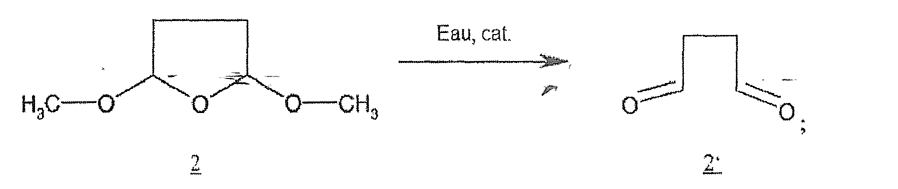
(2) conversion d'un composé de formule 1' et d'un composé de formule 2' avec une solution de R¹-amine en un composé de formule 3 : 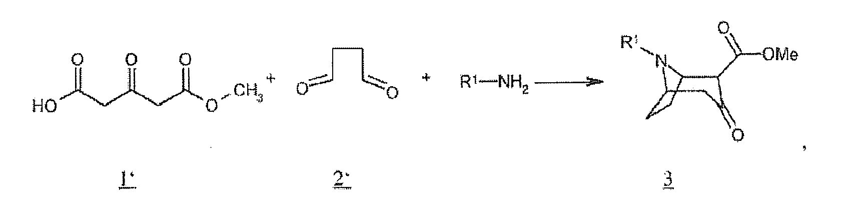 dans laquelle R¹ est un atome d'hydrogène, un radical alkyle, de préférence méthyle, éthyle, propyle ou butyle ou un groupe protecteur quelconque, de préférence allyle, benzyle, méthoxybenzyle, allyloxycarbonyle, benzyloxycarbonyle, tert-butyloxycarbonyle, 9-fluorénylméthyloxycarbonyle, acétyle, benzoyle ou formyle ;
(3) conversion éventuelle du composé de formule 3 avec de l'acide (2R, 3R)-tartrique [acide L(+)-tartrique] avec enrichissement du sel (R)-énantiomère-hydrogénotartrate du composé de formule 3' : 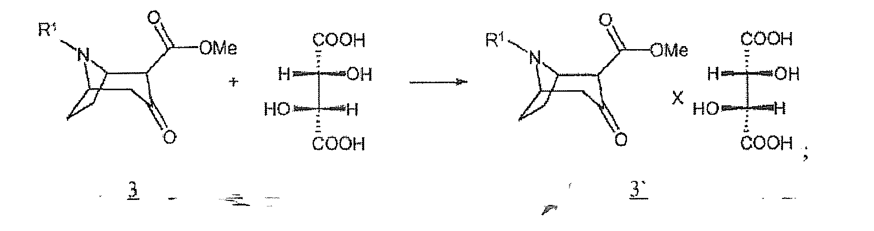
(4) libération éventuelle du composé 4 dans le composé basique 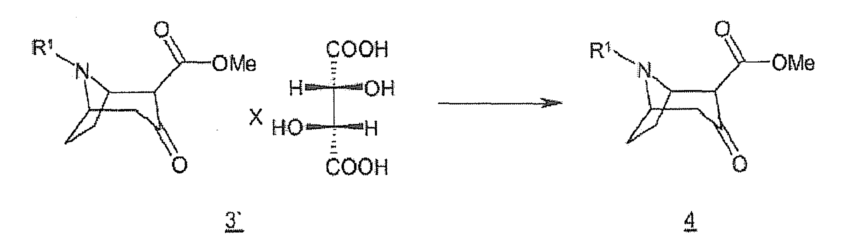
(5) réduction du produit obtenu et éventuellement nouvelle conversion avec un alcoolat de formule MOR² en un composé de formule 5 : 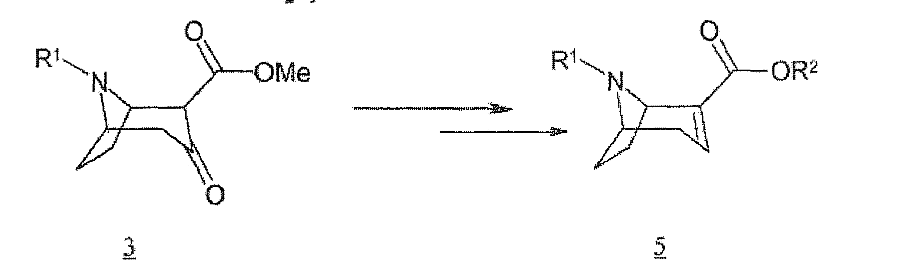 dans laquelle R² est un groupe alkyl, aryle, de préférence phényle ou naphtyle, éventuellement substitué par un ou plusieurs substituants choisis parmi un atome d'halogéne, un groupe hydroxy, amino, cyano, nitro, trifluorométhyle, trifluorométhoxy, alcoxy, cycloalcoxy, alkyle, cycloalkyle, cycloalkylalkyle, alcényle et alcinyle, R² est de préférence un groupe méthyle, éthyle, propyle ou butyle, M est un métal alcalin ou alcalino-terreux, de préférence de potassium ou le sodium ; et
(6) conversion du composé de formule 5 avec un acide chiral en un sel du composé de formule 5' : 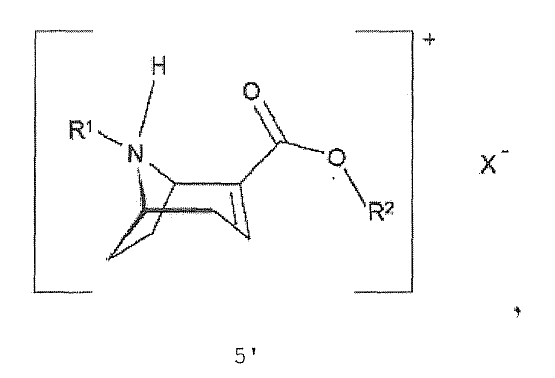 où X⁻ est l'anion d'un acide chiral qui est choisi de préférence dans le groupe comprenant l'acide (1S, 4R)-camphre-10-sulfonique, l'acide (1R, 4S)-camphre-10-sulfonique, l'acide (2R, 3R)-di-p-toluoyl-tartrique, l'acide (2S, 3S)-di-p-toluyoyltartrique, l'acide (2R, 3R)-tartrique, l'acide (2S, 3S)-tartrique, l'acide (2R, 3R)-dibenzoyltartrique et l'acide (2S, 3S)-dibenzoyltartrique ;
(7) éventuellement, purification du composé chiral de formule 5' et
(8) éventuellement, cristallisation.

2. Procédé selon la revendication 1, **caractérisé en ce que**, à la fin du traitement à l'étape (5), par addition de toluène, une solution concentrée toluolique du composé de formule 5 est produite.

3. Procédé selon la revendication 1, **caractérisé en ce qu'**à l'étape (6), le composé de formule 5 se présente sous forme de concentré dissous dans du toluène, à au moins 20 % en poids, de préférence au moins 40 % en poids, de manière particulièrement préférée au moins 60 % en poids.

4. Procédé selon la revendication 1, **caractérisé en ce qu'**à l'étape (6), l'acide chiral est soumis à un solvant et à cette fin, la solution toluolique concentrée du composé de formule 5 est ajoutée.

5. Procédé selon la revendication 1, **caractérisé en ce que** le solvant à l'étape (6) est choisi parmi les solvants protiques ou aprotiques, polaires ou semi-polaires, de préférence l'acétone, les alcools en C₁ à C₅, les nitriles en C₂ à C₃, les cétones en C₃ à C₆ avec ou sans adjonction d'eau.

6. Procédé selon la revendication 1, **caractérisé en ce que** l'acide chiral est dissous dans le solvant de l'étape (6) par chauffage dans une plage de températures d'environ 35 °C à environ la température de reflux du solvant utilisé, le composé de formule 5 est ajouté sous la forme d'une solution ou suspension toluolique concentrée de la solution d'acide chiral à la température de dissolution ou à une température proche de celle-ci, de l'acide chiral, après addition de la solution ou suspension toluolique concentrée, et éventuellement, chauffage jusqu'à la température de reflux du solvant, le mélange est refroidi pour précipiter le composé de formule 5', de préférence jusqu`à une température finale entre -15 et 35 °C, de préférence de 5 à 35 °C et le processus de précipitation est favorisé éventuellement par inoculation d'une petite quantité de cristaux d'inoculation et/ou trituration.

7. Procédé de production de sels du composé de formule 5 : 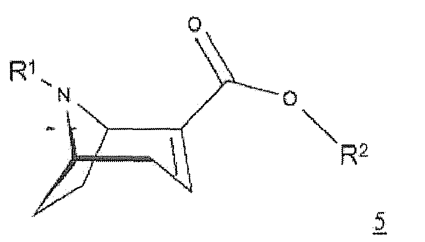 dans lequel le composé de formule 5 est converti avec un acide chiral en un sel du composé de formule 5' : 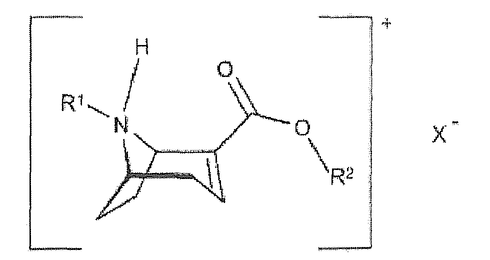 dans lequel R¹ et R² sont tels que définis dans la revendication 1, et
X est l'anion d'un acide chiral qui est choisi de préférence dans le groupe comprenant l'acide (1S, 4R)-camphre-10-sulfonique, l'acide (1R, 4S)-camphre-10-sulfonique, l'acide (2R, 3R)-di-p-toluoyl-tartrique, l'acide (2S, 3S)-di-p-toluyoyltartrique, l'acide (2R, 3R)-tartrique, l'acide (2S, 3S)-tartrique, l'acide (2R, 3R)-dibenzoyltartrique et l'acide (2S, 3S)-dibenzoyltartrique.

8. Procédé selon la revendication 7, **caractérisé en ce que** les conditions correspondent à celles selon l'une quelconque des revendications 2 à 6.

9. Procédé selon au moins l'une quelconque des revendications précédentes 7 ou 8, **caractérisé en ce que** le composé de formule 5' est précipité sous la forme d'un énantiomère, en un motif énantiomère de plus d'environ 95 %, de préférence de plus d'environ 96 %, de manière particulièrement préférée de plus d'environ 98 %, en particulier de plus d'environ 99 %, de manière tout particulièrement préférée, de plus d'environ 99,9 %.

10. Sel sans énantiomère du composé de formule 5 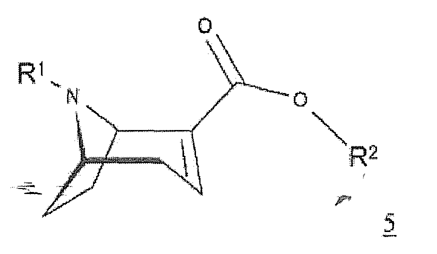 dans laquelle
R¹ est un radical alkyle, de préférence un groupe méthyle, éthyle, propyle ou butyle ou un groupe protecteur quelconque, de préférence allyle, benzyle, méthoxybenzyle, allyloxycarbonyle, benzyloxycarbonyle, tert-butyloxycarbonyle, 9-fluorénylméthyloxycarbonyle, acétyle, benzoyle ou formule ;
R² est un groupe alkyle, aryle, de préférence phényle ou naphtyle, éventuellement substitué par un ou plusieurs substituants choisis parmi un atome d'halogène, un groupe hydroxy, amino, cyano, nitro, trifluorométhyle, trifluorométhoxy, alcoxy, cycloalcoxy, alkyle, cycloalkyle, cycloalkylalkyle, alcényle et alcinyle ; de préférence R² est un groupe méthyle, éthyle, propyle ou butyle,
avec un acide chiral qui n'est pas le (1R, 5S)-anhydroecgonine-éthylester-(2'-S, 3'S)-dibenzoyl-hydrogénotartrate.

11. Sel chiral, de préférence sans énantiomère, du composé de formule 5 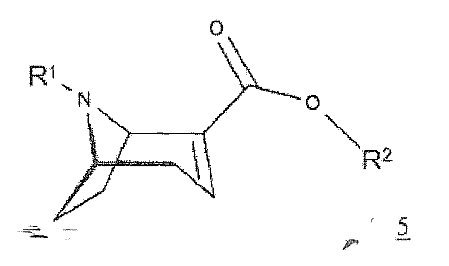 dans laquelle
R¹ est un radical alkyle, de préférence un groupe méthyle, éthyle, propyle ou butyle ou un groupe protecteur quelconque, de préférence allyle, benzyle, méthoxybenzyle, allyloxycarbonyle, benzyloxycarbonyle, tert-butyloxycarbonyle, 9-fluorénylméthyloxycarbonyle, acétyle, benzoyle ou formyle ;
R² est un groupe alkyle, aryle, de préférence phényle ou naphtyle, éventuellement substitué par un ou plusieurs substituants choisis parmi un atome d'halogène, un groupe hydroxy, amino, cyano, nitro, trifluorométhyle, trifluorométhoxy, alcoxy, cycloalcoxy, alkyl, cycloalkyl, cycloalkylalkyle, alcényle et alcinyle ; de préférence R² est un groupe méthyle, éthyle, propyle ou butyle,
avec un acide chiral qui n'est pas le (1R, 5S)-anhydroecgonine-éthylester-(2'-S, 3'S)-dibenzoyl-hydrogénotartrate.

12. Sel selon la revendication 10 ou 11, **caractérisé en ce que** l'acide chiral est choisi parmi l'acide (1S, 4R)-camphre-10-sulfonique, l'acide (1R, 4S)-camphre-10-sulfonique, l'acide (2R, 3R)-di-p-toluoyl-tartrique, l'acide (2S, 3S)-di-p-toluyoyltartrique, l'acide (2R, 3R)-tartrique, l'acide (2S, 3S)-tartrique, l'acide (2R, 3R)-dibenzoyltartrique et l'acide (2S, 3S)-dibenzoyltartrique et le composé de formule 5 est choisi parmi le (1R, 5S)-anhydroecgonine-éthylester, le (1R, 5S)-anhydroecgonineméthylester, le (1R, 5S)-anhydroecgoninpropylester et le (1R, 5S)-anhydroecgoninebutylester.

13. Sel selon l'une quelconque des revendications 10 à 12, **caractérisé en ce que** le sel représente l'un des composés suivants :
(1R, 5S)-anhydroecgonineéthylester-(1'S, 4'R)-camphre-10-sulfonate ;
(1R, 5S)-anhydroecgonineéthylester-(1'R, 4'S)-camphre-10-sulfonate ;
(1R, 5S)-anhydroecgonineéthylester-(2'S, 3'S)-di-p-toluoylhydrogénotartrate ;
1R, 5S)-anhydroecgonineéthylester-(2'R, 3'R)-di-p-toluoylhydrogénotartrate ;
(1R,5S)-anhydroecgonineéthylester-(2'R, 3'R)-hydrogénotartrate-monohydrate ;
(1R, 5S)-anhydroecgonineéthylester-(2'S, 3'S)-hydrogénotartrate-monohydrate ;
(1R, 5S)-anhydroecgonineéthylester-(2'R, 3'R)-hydrogénotartrate ;
(1R, 5S)-anhydroecgonineéthylester-(2'S, 3'S)-hydrogénotartrate ;
(1R, 5S)-anhydroecgonineéthylester-(2'R, 3'R)-dibenzoylhydrogénotartrate,
(1R, 5S)-anhydroecgonineéthylester-(2'S, 3'S)-dibenzoylhydrogénotartrate, de préférence (1R, 5S)-anhydroecgonineéthylester-(1'S, 4'R)-camphre-10-sulfonate ;
(1R,5S) -anhydroecgonineéthylester- (1'R, 4'S)-camphre-10-sulfonate ;
(1R, 5S) -anhydroergonineéthylester- (2'S,3'S) -di-p-toluoylhydrogénotartrate ;
(1R, 5S)-anhydroecgonineéthylester-(2'R, 3'R)-di-p-toluoylhydrogénotartrate ;
(1R, 5S)-anhydroecgonineéthylester-(2'R, 3'R)-hydrogénotartrate-monohydrate ;
(1R, 5S)-anhydroecgonineéthylester-(2'S, 3'S)-hydrogénotartrate-monohydrate ;
(1R, 5S)-anhydroecgonineéthylester-(2'R, 3'R)-hydogénotartrate ;
(1R, 5S)-anhydroecgonineéthylester-(2'S, 3'S)-hydrogénotartrate ;
(1R, 5S)-anhydroecgonineéthylester-(2'R, 3'R)-dibenzoylhydrogénotartrate.

14. Solution d'un (1R, 5S)-anhydroecgonine-ester de formule chimique générale 5 selon la revendication 1, présentant une teneur d'au moins 20 % en poids, de préférence d'au moins 40 % en poids et de manière davantage préférée d'au moins 60 % en poids dans du toluène, du xylène (tous les isomères), des halogénobenzènes, des hydrocarbures aliphatiques (en C₅ à C₈), des hydrocarbures aliphatiques (en C₁ à C₆) contenant un halogène, des éthers aliphatiques (en C₄ à C₈) , des esters d'acide formique (en C₂ à C₇), des esters d'acide acétique (en C₃ à C₇) ou des nitriles (en C₂ à C₅), de préférence, le toluène.

15. Suspension consistant en un agent de mise en suspension choisi dans le groupe comprenant le toluène, le xylène (tous les isomères), des halogénobenzènes, des hydrocarbures aliphatiques (en C₅ à C₈) , des hydrocarbures aliphatiques (en C₁ à C₆) contenant un halogène, des éthers aliphatiques (en C₄ à C₈), des alkylesters d'acide formique (en C₂ à C₇), des esters d' acide acétique (en C₃ à C₇) ou des alkyl-nitriles (en C₂ à C₅), de préférence le toluène et un sel selon l'une quelconque des revendications 10 à 13.
